# EUROPEAN PATENT APPLICATION

(11) **EP 3 549 950 A1**
(43) Date of publication of application: **09.10.2019**
(21) Application number: 17879430.1
(22) Date of filing: 05.12.2017
(51) Int. Cl.: C07K 14/47, C07K 16/18, C07K 16/28, A61K 47/68

(54) **CONJUGATE HAVING ATTENUATED IMMUNE RESPONSE**

(30) Priority: 05.12.2016 KR 20160164619
(71) Applicant: Hanmi Pharm. Co., Ltd., Hwaseong-si, Gyeonggi-do 18536 (KR)
(72) Inventor: KIM, Jin Young, Hwaseong-si Gyeonggi-do 18469 (KR); LEE, Jong Soo, Hwaseong-si Gyeonggi-do 18469 (KR); CHOI, In Young, Hwaseong-si Gyeonggi-do 18469 (KR); JUNG, Sung Youb, Hwaseong-si Gyeonggi-do 18469 (KR)
(74) Representative: Mewburn Ellis LLP
(86) International application number: PCT/KR2017/014139
(87) International publication number: WO 2018/105988

(57) **Abstract**

The present invention relates to a conjugate of a physiologically active polypeptide and immunoglobulin Fc with attenuated immune response, a method for preparing the conjugate, a composition for reducing an immune response including the conjugate, and a method for reducing the immune response of the physiologically active polypeptide. Further, the present invention relates to a method for maintaining the reduction in the intrinsic binding affinity of the conjugate for an Fc gamma receptor and/or a complement, and a composition including the conjugate.

## Description

### [Technical Field]

The present invention relates to a conjugate of a physiologically active polypeptide and immunoglobulin Fc with attenuated immune response, a method for preparing the conjugate, a composition for reducing an immune response including the conjugate, and a method for reducing the immune response of the physiologically active polypeptide.

Further, the present invention relates to a method for maintaining the reduction in the intrinsic binding affinity of the conjugate for an Fc gamma receptor and/or a complement, and a composition including the conjugate.

### [Background Art]

Various protein therapeutics have a disadvantage in that their serum half-life is too short, and thus it is necessary to increase the administration interval or dosage. In this regard, various studies have been conducted on protein conjugates or complexes to which a carrier, such as polyethylene glycol, albumin, fatty acid or antibody Fc region, is linked in order to increase the serum half-life of the proteins. Studies known to date on such protein conjugates or complexes mostly aim to increase the serum half-life of a drug so as to shorten the interval of drug administration to thereby improve patient convenience. However, many conventional technologies have problems such as a decrease in the activity of a protein due to, for example, a spatial hindrance caused by a non-specific binding between a therapeutic protein and a carrier protein. In addition, in the case of fatty acid conjugates that reversibly bind to serum albumin to increase their serum half-life, there is a limitation in significantly increasing the serum half-life, because renal clearance, which accounts for the greatest loss of a protein drug, cannot be avoided due to the reversible binding between the protein and the fatty acid.

Meanwhile, protein therapeutics have problems, in addition to the problem on the increase in serum half-life, that they cause unwanted immune responses due to the immunogenicity of protein therapeutics themselves and thus it is difficult to predict the treatment modality in patient, and further, undesirable immune responses are induced, which result in reduced efficacy of protein therapeutics, anaphylaxis and occasionally life-threatening autoimmunity (Self Nonself. 2010 Oct-Dec; 1(4): 314-322).

Additionally, efforts have been made to use immunoglobulin fragments to increase the half-life of physiologically active substances including proteins. However, the CH2-CH3 region of an immunoglobulin Fc includes a region functioning as an immune effector by binding to an intrinsic Fc gamma receptor and/or a complement of an antibody. The Fc fragments of a human antibody bind to an Fc gamma receptor and a complement thereof to induce the antibody-dependent cell-mediated cytotoxicity (ADCC) and the complement-dependent cytotoxicity (CDC), thereby activating immune functions against antigens. The Fc gamma receptors are known to be involved in the acquired immune response in various ways, in addition to the antibody-dependent cell-mediated cytotoxicity, which is the innate immune response. Specifically, the Fc gamma receptors function in maturation of dendritic cells (DCs) in response to an antigen-antibody complex, antigen presentation and the regulation of B-lymphocyte activation and plasma-cell survival. Further, the Fc gamma receptors are involved in regulating the production and specificity of antibodies, and by regulating the activity of dendritic cells, they serves to distinguish an immunogenic or tolerogenic response after the recognition of antigen peptides (Nature review Immunology, 2008, 8:34-47). The Fc gamma receptors I and IIA are mainly distributed in monocytes, dendritic cells, macrophages and granulocytes, and the Fc gamma receptors IIC and IIIA are mainly distributed in NK cells (natural killer cell). The Fc gamma receptor IIIB is distributed in granulocytes and induce the immune response (Nature review Immunology, 2010, 10: 328-343). Meanwhile, the Fc gamma receptor IIB is widely distributed in various types of lymphocytes, myelocytes, and granulocytes except NK cells and T lymphocytes. Unlike other types of receptors, it inhibits B-lymphocyte activation and humoral immune response, thereby functioning to suppress excessive immune response such as autoimmune response (Nature review Immunology, 2008, 8:34-47).

Accordingly, when the immunoglobulin Fc fragment is used as a carrier for increasing the serum half-life of an physiologically active substance, the half-life may be increased by linking the immunoglobulin Fc fragment to a physiologically active substance, but it is required to develop a method for preventing an immune response from being activated by the immunoglobulin Fc fragment.

Moreover, it is required to develop a method for preventing an unnecessary immune response caused by a physiologically active substance while maintaining the therapeutic effect of an API (active pharmaceutical ingredient) showing the therapeutic effect, *i.e*., the physiologically active substance itself.

### [Disclosure]

### [Technical Problem]

An object of the present invention is to provide a conjugate of a physiologically active polypeptide-immunoglobulin Fc fragment with attenuated immune response.

The conjugate may be a conjugate of a physiologically active polypeptide-immunoglobulin Fc fragment, in which an immunoglobulin Fc fragment is linked to an physiologically active polypeptide, which is characterized in that its immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

The conjugate may be a conjugate of a physiologically active polypeptide-immunoglobulin Fc fragment having the following features of (a), (b), or both, in which an physiologically active polypeptide is linked to an immunoglobulin Fc fragment whose immune response is attenuated as compared to a human serum-derived immunoglobulin G or an Fc fragment thereof: wherein
(a) the conjugate exhibits an attenuated immune response compared to an immune response caused by the physiologically active polypeptide alone; and
(b) the conjugate exhibits a corresponding or reduced immune response compared to an immune response of the immunoglobulin Fc fragment itself.

Another object of the present invention is to provide a conjugate of a physiologically active polypeptide-immunoglobulin Fc fragment, in which a physiologically active polypeptide is linked to an immunoglobulin Fc fragment with an attenuated immune response.

The attenuation of the immune response may be characterized in that the immune response is attenuated as compared to a human serum-derived immunoglobulin G or an Fc fragment thereof.

The conjugate of the physiologically active polypeptide-immunoglobulin Fc fragment may be characterized in that the intrinsic binding affinity of an immunoglobulin Fc fragment for an intrinsic Fc gamma receptor or a complement is significantly reduced.

The conjugate of the physiologically active polypeptide-immunoglobulin Fc fragment may be characterized in that the binding affinity for an Fc gamma receptor I, IIIA and/or complement 1q (C1q) is significantly reduced as compared to a human serum-derived immunoglobulin G or an Fc fragment thereof.

The binding affinity of the conjugate of the physiologically active polypeptide-immunoglobulin Fc fragment for an Fc gamma receptor I, IIIA and/or complement 1q (C1q) may be reduced by 90% or less, 80% or less, 70% or less, 60% or less, 50% or less, 40% or less, 30% or less, 20% or less, 10% or less, 5% or less, as compared to a human serum-derived immunoglobulin G or an Fc fragment thereof.

Still another object of the present invention is to provide a composition for reducing an immune response, including the conjugate of the physiologically active polypeptide-immunoglobulin Fc fragment, wherein the reduction of the immune response is characterized in that the immune response is attenuated as compared to an immune response caused by each the immunoglobulin Fc fragment or the physiologically active polypeptide alone.

Still another object of the present invention is to provide a method for preparing the conjugate of the physiologically active polypeptide-immunoglobulin Fc fragment, including:
(a) preparing a conjugate mixture of a physiologically active polypeptide-immunoglobulin Fc fragment by linking a physiologically active polypeptide to an immunoglobulin Fc fragment via a non-peptidyl linker; and
(b) separating the conjugate whose immune response is attenuated as compared to the physiologically active polypeptide or immunoglobulin Fc fragment.

Still another object of the present invention is to provide a method for preparing a conjugate of a physiologically active polypeptide-immunoglobulin Fc fragment, in which the immune response of the immunoglobulin Fc fragment is attenuated, including:
(a) preparing a conjugate mixture of a physiologically active polypeptide-immunoglobulin Fc fragment by linking a physiologically active polypeptide to an immunoglobulin Fc fragment with an attenuated immune response via a non-peptidyl linker; and
(b) separating the conjugate whose immune response is attenuated as compared to a serum-derived immunoglobulin G.

Still another object of the present invention is to provide a method for reducing the immune response of a conjugate of a physiologically active polypeptide-immunoglobulin Fc fragment by linking a physiologically active polypeptide to an immunoglobulin Fc fragment via a non-peptidyl linker, wherein the reduction of the immune response is characterized in that the immune response is attenuated as compared to an immune response caused by each of the immunoglobulin Fc fragment or the physiologically active polypeptide alone.

Still another object of the present invention is to provide a method for maintaining a reduced binding affinity of a conjugate of a physiologically active polypeptide-immunoglobulin Fc fragment for a Fc gamma receptor and a complement as compared to a human serum-derived immunoglobulin G or a fragment thereof, by linking a physiologically active polypeptide to an immunoglobulin Fc fragment, whose binding affinity for an Fc gamma receptor and a complement is removed, via a linker.

Still another object of the present invention is to provide a composition including the conjugate of the physiologically active polypeptide-immunoglobulin Fc fragment, in which the intrinsic binding affinity of the immunoglobulin Fc fragment for an Fc gamma receptor or complement is reduced. The significant reduction in the intrinsic binding affinity of the immunoglobulin Fc fragment for an Fc gamma receptor or complement may be characterized in that the function of an intrinsic immune effector of the immunoglobulin Fc fragment is significantly reduced.

### [Technical Solution]

In one aspect to overcome the objects above, the present invention provides a conjugate of a physiologically active polypeptide-immunoglobulin Fc fragment with attenuated immune response, in which a physiologically active polypeptide is linked to an immunoglobulin Fc fragment.

In one embodiment, the conjugate may be characterized in that its immune response is attenuated as compared to an immune response caused by each of the physiologically active polypeptide or the immunoglobulin Fc fragment alone.

As the conjugate according to the aforementioned embodiment, the conjugate of the physiologically active polypeptide-immunoglobulin Fc fragment may reduce the immune response caused by the physiologically active polypeptide or the immunoglobulin Fc fragment, each of which is a moiety constituting the conjugate.

As the conjugate according to any one of the aforementioned embodiments, the immune response may be triggered by T-cell proliferation or secretion of IL-2 (Interleukin-2) by T cells of the immunoglobulin Fc fragment, physiologically active polypeptide, or conjugate of the physiologically active polypeptide-immunoglobulin Fc fragment. Herein, the immune response may be attenuated as compared to the level of immune response exhibited by the physiologically active polypeptide or immunoglobulin Fc fragment alone.

As the conjugate according to any one of the aforementioned embodiments, the conjugate is characterized in that a physiologically active polypeptide is linked to an immunoglobulin Fc fragment whose immune response is attenuated as compared to a human serum derived-immunoglobulin G or a fragment thereof. In particular, the attenuation of the immune response may be characterized in that the immune response is attenuated as compared to a human serum derived-immunoglobulin G or a fragment thereof.

As used herein, the attenuation of the immune response of the conjugate of the physiologically active polypeptide-immunoglobulin Fc fragment means that the intensity of the immune response caused by the corresponding physiologically active polypeptide is attenuated as compared to a physiologically active polypeptide, but is not particularly limited thereto.

The immune response caused by any physiologically active polypeptide can be measured by conventional methods known in the art. For example, a method of measuring T cell proliferation or IL-2 secretion by cells using the EpiScreen assay may be used.

As used herein, the attenuation of the immune response of an immunoglobulin Fc fragment means that the intensity of an immune response caused by the immunoglobulin Fc fragment is attenuated as compared to an immune response caused by a human serum derived-immunoglobulin G or a fragment thereof.

The immune response caused by any immunoglobulin Fc fragment can be measured by an Fc gamma receptor binding assay, which is a conventional method known in the art. For example, it may be measured by an ELISA experiment in which the degree of binding affinity is confirmed by an O.D value (450 nm) by adding immunoglobulin Fc fragments diluted with various concentrations to a plate coated with an Fc receptor, and by an SPR experiment which analyzes the binding affinity (KD=Kd/Ka) that can be calculated from the receptor binding constant (Ka) and dissociation constant (Kd) by immobilizing Fc receptors on a CM5 sensor chip and allowing immunoglobulin G or immunoglobulin Fc fragments diluted with various concentrations to flow into the Fc receptors. Further, the conventional methods known in the art, such as FRET (fluorescence resonance energy transfer), BRET (bioluminescence resonance energy transfer), AlphaScreen™ (Amplified Luminescent Proximity Homogeneous Assay), scintillation proximity assay, isothermal titration calorimetry, differential scanning calorimetry, gel electrophoresis, chromatography including gel-filtration chromatography or the like, may be used.

As the conjugate according to any one of the aforementioned embodiments, the conjugate of the physiologically active polypeptide-immunoglobulin Fc fragment is characterized in that T-cell proliferation or IL-2 secretion by T cells is reduced. The reduction of the T-cell proliferation or IL-2 secretion by T cells may be characterized in that the T-cell proliferation or IL-2 secretion by T cells is reduced by 90% or less, 80% or less, 70% or less, 60% or less, 50% or less, 40% or less, 30% or less, 20% or less, 10% or less, 5% or less, as compared to the physiologically active polypeptide or the immunoglobulin Fc fragment.

As the conjugate according to any one of the aforementioned embodiments, the immunoglobulin Fc fragment is characterized in that the binding affinity for an Fc gamma receptor is reduced. The reduction of the binding affinity may be characterized in that the binding affinity for an Fc gamma receptor is reduced by 90% or less, 80% or less, 70% or less, 60% or less, 50% or less, 40% or less, 30% or less, 20% or less, 10% or less, 5% or less, as compared to the physiologically active polypeptide or immunoglobulin Fc fragment.

As the conjugate according to any one of the aforementioned embodiments, the immunoglobulin Fc fragment is characterized in that the binding affinity for a complement 1q is reduced. The reduction of the binding affinity may be characterized in that the binding affinity for a complement 1a (C1q) is reduced by 90% or less, 80% or less, 70% or less, 60% or less, 50% or less, 40% or less, 30% or less, 20% or less, 10% or less, 5% or less, as compared to the physiologically active polypeptide or immunoglobulin Fc fragment.

As the conjugate according to any one of the aforementioned embodiments, the physiologically active polypeptide may be linked to the immunoglobulin Fc fragment via a non-peptidyl linker.

As the conjugate according to any one of the aforementioned embodiments, the non-peptidyl linker may be selected from the group consisting of polyethylene glycol, polypropylene glycol, an ethylene glycol-propylene glycol copolymer, polyoxytheylated polyol, polyvinyl alcohol, polysaccharide, dextran, polyvinyl ether, a biodegradable polymer, a lipid-polymer, chitin, hyaluronic acid, and a combination thereof.

As the conjugate according to any one of the aforementioned embodiments, the non-peptidyl linker may be a polyethylene glycol polymer represented by Chemical Formula 1 below: 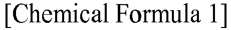 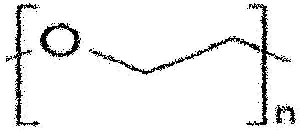 wherein, n = 10 to 2400.

As the conjugate according to any one of the aforementioned embodiments, the reactive group of the non-peptidyl linker may be selected from the group consisting of an aldehyde group, a maleimide group, and a succinimide derivative. The reactive group may be a propionaldehyde group or a butyl aldehyde group.

The physiologically active polypeptide that can be applied to the conjugate according to any one of the aforementioned embodiments may be selected from the group consisting of various physiologically active polypeptides such as hormones, cytokines, interleukins, interleukin-binding proteins, enzymes, antibodies, growth factors, transcription factors, blood factors, vaccines, structural proteins, ligand proteins or receptors, cell surface antigens, or receptor antagonists, and analogs thereof.

As the conjugate according to any one of the aforementioned embodiments, the physiologically active polypeptide may be selected from the group consisting of glucagon-like peptide-1 (GLP-1), granulocyte colony stimulating factor (G-CSF), human growth hormone (hGH), erythropoietin (EPO), glucagon, oxyntomodulin, insulin, growth hormone-releasing hormone, growth hormone-releasing peptide, interferons, interferon receptors, G-protein-coupled receptor, interleukins, interleukin receptors, enzymes, interleukin-binding protein, cytokine-binding protein, macrophage-activating factor, macrophage peptide, B-cell factors, T-cell factors, protein A, allergy inhibitor, cell necrosis glycoprotein, immunotoxin, lymphotoxin, tumor necrosis factor, tumor suppressor, metastasis growth factor, alpha-1 antitrypsin, albumin, α-lactalbumin, apolipoprotein-E, highly-glycosylated erythropoietin, angiopoietins, hemoglobin, thrombin, thrombin receptor-activating peptide, thrombomodulin, blood factors VII, VIIa, VIII, IX and XIII, plasminogen-activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone-stimulating protein, calcitonin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factor, parathyroid hormone, relaxin, secretin, somatomedin, insulin-like growth factor, adrenocortical hormone, cholecystokinin, pancreatic polypeptide, gastrin-releasing peptide, corticotropin-releasing factor, thyroid-stimulating hormone, autotaxin, lactoferrin, myostatin, cell surface antigens, virus-derived vaccine antigens, monoclonal antibodies, polyclonal antibodies, antibody fragments and analogs thereof.

As the conjugate according to any one of the aforementioned embodiments, the enzyme may be selected from the group consisting of imiglucerase, iduronate 2-sulfatase, alpha-galactosidase A, iduronidase (or laronidase), alpha-glucosidase, beta-glucosidase, beta-galactosidase, galactose-6-sulfatase, acid ceramidase, acid sphingomyelinase, galactocerebrosidase, arylsulfatase A, arylsulfatase B (or galsulfase), beta-hexosaminidase A, beta-hexosaminidase B, heparin N-sulfatase, alpha-D-mannosidase, beta-glucuronidase, N-acetylgalactosamine-6 sulfatase, lysosomal acid lipase, alpha-N-acetyl-glucosaminidase (NAGLU), glucocerebrosidase, butyrylcholinesterase, chitinase, glutamate decarboxylase, lipase, uricase, platelet-activating factor acetylhydrolase, neutral endopeptidase, myeloperoxidase, acetyl-CoA-glucosaminide N-acetyltransferase, N-acetylglucosamine-6-sulfatase, galactosamine 6-sulfatase (GALN), hyaluronidase, α-fucosidase, β-mannosidase, α-neuraminidase (sialidase), N-acetyl-glucosamine-1-phosphotransferase, mucolipin-1, α-N-acetyl-galactosaminidase, N-aspartyl-β-glucosaminidase, LAMP-2, cystinosin, sialin, ceramidase, acid-β-glucosidase, galactosylceramidase, NPC1, cathepsin A (protective protein), SUMF-1, lysosomal acid lipase (LIPA), and tripeptidyl peptidase 1.

As the conjugate according to any one of the aforementioned embodiments, the immunoglobulin Fc fragment may include a CH2 domain, a CH3 domain, or both.

As the conjugate according to any one of the aforementioned embodiments, the immunoglobulin Fc fragment may be non-glycosylated.

As the conjugate according to any one of the aforementioned embodiments, the immunoglobulin Fc fragment may further include a hinge region.

As the conjugate according to any one of the aforementioned embodiments, the immunoglobulin Fc fragment may be selected from the group consisting of IgG, IgA, IgD, IgE, IgM, a combination thereof, and a hybrid thereof.

As the conjugate according to any one of the aforementioned embodiments, the immunoglobulin Fc fragment may be an IgG4 Fc fragment.

In another aspect, the present invention provides a composition for reducing an immune response, including the conjugate of the physiologically active polypeptide-immunoglobulin Fc fragment, wherein the reduction of the immune response is characterized in that the immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

In still another aspect, the present invention provides a method for preparing the conjugate of the physiologically active polypeptide-immunoglobulin Fc fragment, including:
(a) preparing a conjugate mixture of a physiologically active polypeptide-immunoglobulin Fc fragment by linking a physiologically active polypeptide to an immunoglobulin Fc fragment via a non-peptidyl linker; and
(b) separating the conjugate whose immune response is attenuated as compared to the physiologically active polypeptide or immunoglobulin Fc fragment.

In one embodiment, the method for preparing the conjugate may be a method for preparing a conjugate of a physiologically active polypeptide-immunoglobulin Fc fragment, including:
(a) preparing a conjugate mixture of a physiologically active polypeptide-immunoglobulin Fc fragment by linking a physiologically active polypeptide to an immunoglobulin Fc fragment with an attenuated immune response via a non-peptidyl linker; and
(b) separating the conjugate whose immune response is reduced as compared to a serum-derived immunoglobulin G.

As the preparation method according to any one of the aforementioned embodiments, the attenuation of the immune response is characterized in that the binding affinity of the immunoglobulin Fc fragment for an Fc gamma receptor and a complement is removed.

As the preparation method according to any one of the aforementioned embodiments, the immunoglobulin Fc fragment with attenuated immune response may be a non-glycosylated Fc fragment.

As the preparation method according to any one of the aforementioned embodiments, the non-peptidyl linker may be a polyethylene glycol polymer represented by Chemical Formula 1 below: 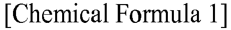 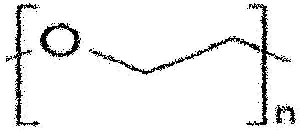 wherein, n = 10 to 2400.

As the preparation method according to any one of the aforementioned embodiments, the step (b) may be for separating a conjugate in a form in which the non-peptidyl linker is linked to the N-terminus of the immunoglobulin Fc fragment.

In still further another aspect, the present invention provides a method for reducing the immune response of a conjugate of a physiologically active polypeptide-immunoglobulin Fc fragment by linking a physiologically active polypeptide to an immunoglobulin Fc fragment via a non-peptidyl linker, wherein the reduction of the immune response is characterized in that the immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

As the method for reducing the immune response according to any one of the aforementioned embodiments, the immune response may be triggered by T-cell proliferation or secretion of IL-2 (Interleukin-2) by T cells of the immunoglobulin Fc fragment, physiologically active polypeptide, or conjugate of the physiologically active polypeptide-immunoglobulin Fc fragment.

In still further another aspect, the present invention provides a method for maintaining a reduced binding affinity of a conjugate of a physiologically active polypeptide-immunoglobulin Fc fragment for an Fc gamma receptor and a complement, by linking a physiologically active polypeptide to an immunoglobulin Fc fragment, whose binding affinity for an Fc gamma receptor and a complement is removed, via a non-peptidyl linker.

In one embodiment, the immunoglobulin Fc fragment, whose binding affinity for an Fc gamma receptor and a complement is removed, may be a non-glycosylated Fc fragment.

In still further another aspect, the present invention provides a composition including the conjugate of the physiologically active polypeptide-immunoglobulin Fc fragment, in which the intrinsic binding affinity of the immunoglobulin Fc fragment for an Fc gamma receptor or complement is reduced as compared to an immunoglobulin G or a fragment thereof.

### [Advantageous Effects]

The conjugate of the present invention can provide a structure of a therapeutic agent capable of attaining a desired therapeutic effect *in vivo* by attenuating an unwanted immune response caused by a protein that has a therapeutic effect in protein therapeutics. In addition, the conjugate of the present invention binds to an immunoglobulin-specific Fc gamma receptor and complement, thereby eliminating the effector function that activates the immune response, and thus does not activate unnecessary immune functions in the body. Therefore, the conjugate can increase the serum half-life of the physiologically active polypeptide and impart safety and thus can be usefully used.

### [Brief Description of Drawings]

FIG. 1 shows the absorbance of the concentration-dependent binding of the immunoglobulin Fc and conjugate for the Fc gamma receptor I (450 nm).
FIG. 2 shows the absorbance of the concentration-dependent binding of the immunoglobulin Fc and conjugate for the Fc gamma receptor IIIA (450 nm).
FIG. 3 shows the absorbance of the concentration dependent binding of the immunoglobulin Fc and conjugate for the complement 1q (C1q) (450 nm).

### [Detailed Description of Embodiments]

Hereinafter, the present invention will be described in more detail. Meanwhile, each description and embodiment disclosed herein can be applied to other descriptions and embodiments, respectively. That is, all combinations of various elements disclosed herein fall within the scope of the present invention. Further, the scope of the present disclosure is not limited by the specific description described below.

In addition, those skilled in the art can recognize and identify numerous equivalents for the specific embodiments of the invention disclosed herein using no more than routine experimentation, and all such equivalents are believed to be within the scope of the invention.

In one aspect of the present invention, there is provided a conjugate of a physiologically active polypeptide-immunoglobulin Fc fragment with attenuated immune response.

The conjugate may be a conjugate of a physiologically active polypeptide-immunoglobulin Fc fragment, in which an immunoglobulin Fc fragment is linked to an physiologically active polypeptide, which is characterized in that its immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

The conjugate may be a conjugate of a physiologically active polypeptide-immunoglobulin Fc fragment having the following features of (a), (b), or both, in which a physiologically active polypeptide is linked to an immunoglobulin Fc fragment whose immune response is attenuated as compared to a human serum-derived immunoglobulin G or an Fc fragment thereof:
(a) the conjugate exhibits an attenuated immune response compared to an immune response caused by a physiologically active polypeptide alone; and
(b) the conjugate exhibits a corresponding or reduced immune response compared to an immune response of the immunoglobulin Fc fragment itself.

More specifically, the conjugate may be a conjugate of a physiologically active polypeptide-immunoglobulin Fc fragment, in which a physiologically active polypeptide is linked to an immunoglobulin Fc fragment whose immune response is attenuated as compared a human serum-derived immunoglobulin G or an Fc fragment thereof, wherein
(a) the conjugate exhibits an attenuated T-cell proliferation, IL-2 secretion by T cells, or both as compared to T-cell proliferation, IL-2 secretion by T cells, or both caused by the physiologically active polypeptide alone, and
(b) the immunoglobulin Fc fragment itself exhibits a reduced binding affinity for an Fc gamma receptor, complement, or both as compared to an immunoglobulin G or an Fc fragment thereof, and the conjugate may be a conjugate that maintains the reduced binding affinity of such immunoglobulin Fc fragment, but is not particularly limited thereto.

In general, an active pharmaceutical ingredient (API), which indicates a drug efficacy in a protein therapeutic, itself acts as an immunogen *in vivo*, causing an undesirable immune response, which poses a risk of unexpected treatment outcome in an administered subject. Surprisingly, however, the conjugate provided by the present inventors can significantly reduce or counteract the immunogenicity exhibited by each of the immunoglobulin Fc fragments that increase the half-life of the physiologically active polypeptide itself or physiologically active polypeptide exhibiting the combined therapeutic effect, and also can maintain the therapeutic effects at the same time and thus can provide a platform for safe protein therapeutics. In particular, the conjugate in which a physiologically active polypeptide is linked to an immunoglobulin Fc fragment whose immune response is attenuated as compared to a human serum derived-immunoglobulin G or a fragment thereof exhibits a reduced (or attenuated) immune response as compared to an immune response exhibited by the physiologically active polypeptide itself, and also maintains the attenuated immune response possessed by the immunoglobulin Fc fragment itself so that the immune response that can be caused by the immunoglobulin Fc fragment is reduced (or attenuated) as compared to a human serum derived-immunoglobulin G or a fragment thereof, even in the form of a conjugate.

The conjugate of the physiologically active polypeptide-immunoglobulin Fc fragment may reduce the immune response caused by the physiologically active polypeptide and the immunoglobulin Fc fragment, each of which is a moiety constituting the conjugate.

As used herein, the term "attenuation of immune response or reduction of immune response" means that the intensity of the immune response (or the frequency of the immune response) exhibited by the conjugate of the physiologically active polypeptide-immunoglobulin Fc fragment is reduced as compared to the intensity of the immune response (or the frequency of the immune response) exhibited by each of the physiologically active polypeptide or immunoglobulin Fc fragment alone.

More specifically, the intensity of the immune response (or the frequency of the immune response) exhibited by the conjugate of the physiologically active polypeptide-immunoglobulin Fc fragment may be reduced compared to the intensity of the immune response (or the frequency of the immune response) exhibited the physiologically active polypeptide alone, but is not particularly limited thereto.

Although not particularly limited, the intensity of the immune response (or the frequency of the immune response) exhibited by the conjugate of the physiologically active polypeptide-immunoglobulin Fc fragment may be 90% or less, 80% or less, 70% or less, 60% or less, 50% or less, 40% or less, 30% or less, 20% or less, 10% or less, 5% or less, as compared to when the intensity of the corresponding immune response (or the frequency of the immune response) (100%) exhibited by each of the physiologically active polypeptide or immunoglobulin Fc fragment alone for a particular type of immune response; or the sum of the intensity of the immune response (or the frequency of the immune response) exhibited by each of the physiologically active polypeptide or immunoglobulin Fc fragment is taken as 100%. More specifically, the intensity of the immune response (or the frequency of the immune response) exhibited by the conjugate of the physiologically active polypeptide-immunoglobulin Fc fragment may be 90% or less, 80% or less, 70% or less, 60% or less, 50% or less, 40% or less, 30% or less, 20% or less, 10% or less, 5% or less, as compared to the intensity of the corresponding immune response (or the frequency of the immune response) (100%) exhibited by the physiologically active polypeptide alone for a particular type of immune response, but is not particularly limited thereto.

The immune response may be triggered by T-cell proliferation or secretion of IL-2 (Interleukin-2) by T cells of the immunoglobulin Fc fragment, physiologically active polypeptide, or conjugate of the physiologically active polypeptide-immunoglobulin Fc fragment. Specifically, the attenuation or reduction of the immune response may be measured by the reduction of T-cell proliferation or reduction of IL-2 secretion by T cells, but is not particularly limited thereto.

Although not particularly limited, when T cell proliferation or IL-2 secretion by T cells is an immune response, the intensity of the immune response (or the frequency of the immune response) exhibited by the conjugate of the physiologically active polypeptide-immunoglobulin Fc fragment may be 90% or less, 80% or less, 70% or less, 60% or less, 50% or less, 40% or less, 30% or less, 20% or less, 10% or less, 5% or less, as compared to the intensity of the corresponding immune response (or the frequency of the immune response) (100%) exhibited by each of the physiologically active polypeptide or immunoglobulin Fc fragment alone. More specifically, the intensity of the immune response (or the frequency of the immune response) exhibited by the conjugate of the physiologically active polypeptide-immunoglobulin Fc fragment may be 90% or less, 80% or less, 70% or less, 60% or less, 50% or less, 40% or less, 30% or less, 20% or less, 10% or less, 5% or less, as compared to the intensity of the corresponding immune response (or the frequency of the immune response) (100%) exhibited by the physiologically active polypeptide alone for a particular type of immune response (in particular, T-cell proliferation or IL-2 secretion by T cells), but is not limited thereto.

The immune response caused by any physiologically active polypeptide can be measured by conventional methods known in the art.

The conjugate may provide a conjugate of a physiologically active polypeptide-immunoglobulin Fc fragment in which a physiologically active polypeptide is linked to an immunoglobulin Fc fragment with attenuated immune response. The conjugate is a substance in which a physiologically active polypeptide is linked to an immunoglobulin Fc fragment with attenuated immune response. As used herein, the phrase "the immune response of any immunoglobulin Fc fragment is attenuated" means that the intensity of an immune response caused by the immunoglobulin Fc fragment is attenuated as compared to an immune response caused by a human serum-derived immunoglobulin G or an Fc fragment thereof.

Meanwhile, the conjugate may exhibit a corresponding or reduced immune response as compared to an immune response of the immunoglobulin Fc fragment itself. Specifically, the conjugate may exhibit a corresponding or reduced immune response as compared to an immune response of the immunoglobulin Fc fragment itself (to a degree that is attenuated compared to a human serum-derived immunoglobulin G or an Fc fragment thereof). That is, the conjugate may maintain the immune response possessed by the immunoglobulin Fc fragment itself. Herein, the immunoglobulin Fc fragment may be characterized in that the binding affinity for an Fc gamma receptor and/or complement is removed. The reduced immune response exhibited by the immunoglobulin Fc fragment may mean that the binding ability for the Fc gamma receptor and/or complement is decreased as compared to a human serum-derived immunoglobulin G or a Fc fragment thereof.

Therefore, as used herein, the phrase "the immune response possessed by the immunoglobulin Fc fragment is maintained" means that even when the immunoglobulin Fc fragment is linked to the physiologically active polypeptide via a non-peptidyl linker, the immune response exhibited by the immunoglobulin Fc fragment still exhibits an immune response corresponding to the immune response exhibited by the immunoglobulin Fc fragment itself. Even more specifically, even when the immunoglobulin Fc fragment, whose binding affinity for an Fc gamma receptor and/or complement is removed, is linked to the physiologically active polypeptide via a non-peptidyl linker, the binding affinity of the immunoglobulin Fc fragment, whose binding affinity for an Fc gamma receptor and/or complement is removed, for an Fc gamma receptor and/or complement which is reduced relative to a human serum-derived immunoglobulin G or a fragment thereof still exhibits a binding affinity for an Fc gamma receptor and/or complement corresponding to the binding affinity exhibited by the immunoglobulin Fc fragment itself, whose binding affinity for an Fc gamma receptor and/or complement is removed, but is not particularly limited thereto.

As used herein, the term "corresponding" means that it shows a difference of at least ± 20%, ± 10%, ± 5%, or 0% compared to the immune response exhibited by the immunoglobulin Fc fragment itself, and even more specifically, it means that it shows a difference of at least ± 20%, ± 10%, ± 5%, or 0% compared to the binding affinity exhibited by the immunoglobulin Fc fragment, whose binding affinity for an Fc gamma receptor and/or complement is removed, for an Fc gamma receptor and/or complement, but is not particularly limited thereto.

Herein, the Fc gamma receptor and/or complement may be Fc gamma receptor I, IIIA and/or complement 1q (C1q), but is not particularly limited thereto.

The immune response caused by any immunoglobulin Fc fragment can be measured by an Fc gamma receptor binding assay, which is a conventional method known in the art. For example, it may be measured by an ELISA experiment in which the degree of binding affinity is confirmed by an O.D value (450 nm) by adding immunoglobulin Fc fragments diluted with various concentrations to a plate coated with an Fc receptor, and by an SPR experiment which analyzes the binding affinity (KD=Kd/Ka) that can be calculated from the receptor binding constant (Ka) and dissociation constant (Kd) by immobilizing Fc receptors on a CM5 sensor chip and allowing immunoglobulin G or immunoglobulin Fc fragments diluted with various concentrations to flow into the Fc receptors. Further, conventional methods known in the art such as, FRET (fluorescence resonance energy transfer), BRET (bioluminescence resonance energy transfer), AlphaScreen™ (Amplified Luminescent Proximity Homogeneous Assay), scintillation proximity assay, isothermal titration calorimetry, differential scanning calorimetry, gel electrophoresis, chromatography including gel-filtration chromatography or the like may be used.

In one specific embodiment, the conjugate of the physiologically active polypeptide-immunoglobulin Fc fragment may be a conjugate of a physiologically active polypeptide and an immunoglobulin Fc fragment in which a physiologically active polypeptide is linked to an immunoglobulin Fc fragment having a significantly reduced immune effector function via a non-peptidyl linker. More specifically, the conjugate of the physiologically active polypeptide-immunoglobulin Fc fragment may provide a conjugate having a binding affinity for an Fc gamma receptor I, IIIA and/or complement 1q (C1q) significantly lower relative to serum IgG.

The binding affinity of the immunoglobulin Fc fragment for an Fc gamma receptor I, IIIA and/or complement 1q may be measured by the ELISA method commonly used in the art by diluting human serum-derived immunoglobulin G or immunoglobulin fragments with various concentrations under a condition of 50 mM sodium carbonate buffer (pH 9.0) and may be measured by the SPR method commonly used in the art under a condition of HBS-EP buffer (10 mM HEPES, 150 mM sodium chloride, 3 mM ethylene diamine acetic acid (EDTA), 0.005% polysorbate 20) at pH 7.4. Specifically, the conjugate may be a conjugate of a physiologically active polypeptide-immunoglobulin Fc fragment, in which a physiologically active polypeptide is linked to an immunoglobulin Fc fragment showing a decrease in the binding affinity for an Fc gamma receptor IIIA and/or complement 1q (C1q) by 90% or less, 80% or less, 70% or less, 60% or less, 50% or less, 40% or less, 30% or less, 20% or less, 10% or less, 5% or less, as compared to a human serum-derived immunoglobulin G or a fragment thereof. Specifically, the decrease in the binding affinity may be characterized in that the binding affinity of the conjugate of the physiologically active polypeptide-immunoglobulin Fc fragment, in which a physiologically active polypeptide is linked to an immunoglobulin Fc fragment, for an Fc gamma receptor I, IIIA and/or complement 1q (C1q) is significantly reduced by 90% or less, 80% or less, 70% or less, 60% or less, 50% or less, 40% or less, 30% or less, 20% or less, 10% or less, 5% or less, compared to a human serum-derived immunoglobulin G or a fragment thereof.

Specifically, the conjugate of the present invention may be a conjugate of a physiologically active polypeptide-immunoglobulin Fc fragment, in which a physiologically active polypeptide is linked to an immunoglobulin Fc fragment showing a significant decrease in the intrinsic binding affinity for an Fc gamma receptor or complement. Accordingly, the present invention provides a conjugate showing a significant decrease in the binding affinity for a complement 1q (Clq) relative to a serum-derived immunoglobulin G (IVIgG) using an enzyme-linked immunosorbent assay (ELISA). With respect to the binding affinity for the Fc gamma receptor I and Fc gamma receptor IIIA, the present invention also provides a conjugate that hardly binds to the Fc gamma receptor I and Fc gamma receptor IIIA relative to serum-derived immunoglobulin G (IVIgG).

In the present invention, it was found that, when a physiologically active polypeptide was covalently linked to an immunoglobulin Fc fragment showing a significant decrease in the binding affinity for an Fc gamma receptor or complement via a non-peptidyl linker to form a conjugate, the low binding affinity for an Fc gamma receptor or complement could be maintained. In particular, when the non-peptidyl linker binds to the immunoglobulin Fc fragment, it has no effect on the reduced binding affinity for the Fc gamma receptor and complement, and when the non-peptidyl linker is polyethylene glycol, wherein n in -[O-CH₂-CH₂]n- is 10 or greater, in particular 50, it was confirmed that it has no effect on the physiological activity of the physiologically active polypeptide and the Fc region and on the suppressed intrinsic binding affinity for each of the Fc gamma receptor and complement, and the present invention has been implement based on these findings.

As used herein, the term "conjugate of the physiologically active polypeptide-immunoglobulin Fc fragment" may be interchangeably used with "a conjugate of a physiologically active polypeptide-immunoglobulin Fc fragment with significantly reduced immune effector function, "a conjugate of a physiologically active polypeptide-immunoglobulin Fc fragment with attenuated immune response" or "a long-acting conjugate".

The conjugate may be a conjugate of a physiologically active polypeptide-immunoglobulin Fc fragment, in which a physiologically active polypeptide is covalently linked to an immunoglobulin Fc fragment via a non-peptidyl linker, which is characterized in that its immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

The conjugate is also characterized in that the binding affinity for the complement 1q (C1q) is remarkably reduced as compared to a serum-derived immunoglobulin G (IVIgG), and with respect to the binding affinity for the Fc gamma receptor I and the Fc gamma receptor IIIA, it hardly binds to the Fc gamma receptor I and the Fc gamma receptor IIIA, as compared to a serum-derived immunoglobulin G (IVIgG).

The non-peptidyl linker in the conjugate may be linked to amino acid residues apart from the FcRn-binding region of the immunoglobulin Fc fragment, for example, a region corresponding to positions 252 to 257 and 307 to 311 of CH2 and positions 433 to 436 of CH3 (numbered according to the Kabat numbering system). For example, the non-peptidyl linker of the present invention may be linked to the N-terminus or C-terminus of the immunoglobulin Fc fragment, and specifically linked to the N-terminus, but is not limited thereto.

As used herein, the term "N-terminus" refers to the amino terminal of a peptide, and refers to a position capable of binding to a linker including a non-peptidyl polymer for the purpose of the present invention. Examples include, but are not limited to, all the amino acid residues around the N-terminal as well as the terminal amino acid residue at the N-terminus, and may specifically include the first to twentieth amino acid residues from the terminal amino acid.

When the non-peptidyl linker of the present invention is linked to the N-terminus or C-terminus of the immunoglobulin Fc, it has no effect on the reduced binding affinity for the Fc gamma receptor and the complement, and thus the suppressed intrinsic binding affinity of the immunoglobulin Fc fragment for the Fc gamma receptor and the complement can be maintained even in the form of a conjugate.

As used herein, the term "non-peptidyl linker" refers to a biocompatible polymer composed of two or more repeating units linked, in which the repeating units are linked to each other by any non-peptide covalent bond. Such a non-peptidyl linker may have two or three ends.

The non-peptidyl linker that can be used in the present invention may be selected from the group consisting of a biodegradable polymer such as polyethylene glycol, polypropylene glycol, a copolymer of ethylene glycol with propylene glycol, polyoxyethylated polyol, polyvinyl alcohol, polysaccharide, dextran, polyvinyl ethyl ether, polylactic acid (PLA) and polylactic-glycolic acid (PLGA), lipid polymer, chitin, hyaluronic acid, and a combination thereof, but is not limited thereto. Specifically, the non-peptidyl linker is polyethylene glycol, for example, a polyethylene glycol polymer represented by the following Chemical Formula 1, but is not limited thereto. 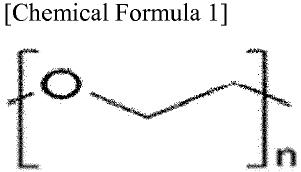
wherein, n = 10 to 2400,
specifically, n = 10 to 480, and
more specifically, n = 50 to 250, but is not limited thereto.

Meanwhile, other non-peptidyl linkers having a molecular weight corresponding to that of the polyethylene glycol represented by Chemical Formula 1 also fall within the scope of the present invention.

Although not particularly limited, the polymer in the present invention may have a molecular weight in the range of more than 0 to about 100 kDa, specifically about 1 to about 100 kDa, and more specifically about 1 to about 20 kDa.

In addition, the derivatives thereof known in the art and derivatives of the non-peptidyl linker that can be easily prepared in the state of the art also fall within the scope of the present invention
The polyethylene glycol used as the non-peptidyl linker in the present invention has advantages in that it does not result in spatial hindrance between the physiologically active polypeptide and immunoglobulin Fc fragment linked to both ends so that the physiological activity of the physiologically active polypeptide can be maintained, and also has no binding affinity for an Fc gamma receptor and a complement and thereby does not influence the activation of unnecessary immune functions.

A peptidyl linker that is used in a fusion protein prepared by a conventional in-frame fusion method has a disadvantage in that it is easily cleaved by protease *in vivo,* and thus the effect of increasing the serum half-life of an active drug by a carrier cannot be obtained as expected. However, the conjugate using the non-peptidyl linker of the present invention has dramatically overcome this disadvantage. The non-peptidyl linker may be a polymer that has resistance to protease and thereby maintains the serum half-life of the peptide, similar to that of a carrier. Therefore, any non-peptidyl linker may be used in the present invention without limitation, as long as it is a polymer having the above-described function, that is, having resistance to protease *in vivo.*

In addition, as the non-peptidyl linker of the present invention that is linked to the immunoglobulin Fc fragment, not only of one kind of polymer, but also a combination of different kinds of polymers may be used.

Moreover, the non-peptidyl linker used in the present invention may have reactive groups capable of binding to the immunoglobulin Fc fragment and the physiologically active polypeptide.

The reactive groups at both ends of the non-peptidyl polymer may be selected from the group consisting of a reactive aldehyde group, for example, a propionaldehyde group or a butyl aldehyde group, a maleimide group, and a succinimide derivative, but are not limited thereto.

Herein, as the succinimide derivative, succinimidyl valerate, succinimidyl methylbutanoate, succinimidyl methylpropionate, succinimidyl butanoate, succinimidyl propionate, N-hydroxysuccinimide, hydroxy succinimidyl, succinimidyl carboxymethyl, or succinimidyl carbonate may be used, but is not limited thereto.

In particular, when the non-peptidyl linker has a reactive aldehyde group at both ends thereof, a physiologically active polypeptide and an immunoglobulin can effectively bind to both ends of the non-peptidyl linker, respectively, while minimizing non-specific reactions. A final product produced by reductive alkylation via an aldehyde bond is much more stable than that linked via an amide bond. The aldehyde reactive group can selectively bind to the N-terminus at a low pH and can form a covalent bond with a lysine residue at a high pH, for example, at pH 9.0. In particular, the non-peptidyl linker may contain two or more aldehyde groups or have two or more alcohol groups substituted with functional groups including aldehyde.

The reactive groups at both ends of the non-peptidyl linker may be the same or different. For example, one end of the non-peptidyl linker may have a maleimide group, and the other end may have an aldehyde group, a propionaldehyde group, or an alkyl aldehyde group such as butyl aldehyde.

When a polyethylene glycol having hydroxyl reactive groups at both ends is used as the non-peptidyl linker, the hydroxy groups may be activated into various reactive groups by a known chemical reaction. Alternatively, a commercially available polyethylene glycol having a modified reactive group may be used to prepare the conjugate of the present invention.

As used herein, the term "physiologically active polypeptide" collectively refers to polypeptides having any physiological action *in vivo,* which commonly have a polypeptide structure and exhibit various physiological activities. The physiologically active polypeptides include those that function to regulate genetic expression and physiological function and to correct an abnormal condition caused by the lack or excessive secretion of a substance involved in the regulation of functions *in vivo,* and may also include general protein therapeutic agents. In addition, the physiologically active polypeptide is meant to include not only native polypeptides, but also analogs thereof.

In the conjugate of the present invention, the kind and size of the physiologically active polypeptide are not specifically limited, as long as it is a physiologically active polypeptide that can exhibit an increase in the serum half-life by the conjugate structure of the present invention. For example, it may be selected from the group consisting of various physiologically active polypeptides such as hormones, cytokines, interleukins, interleukin-binding proteins, enzymes, antibodies, growth factors, transcription factors, blood factors, vaccines, structural proteins, ligand proteins or receptors, cell surface antigens, or receptor antagonists, and analogs thereof, but is not limited thereto.

In an embodiment of the present invention, conjugates were prepared using various physiologically active polypeptides, including insulin analogs, GLP-1R agonists, and enzymes, which are representative examples of physiologically active polypeptides, and it was found that not only the immune response caused by the polypeptides themselves could be reduced regardless of the kind and size of the physiologically active polypeptides, but also the intrinsic binding affinity of the immunoglobulin Fc fragment itself for an Fc gamma receptor and a complement could be reduced.

The physiologically active polypeptide may be selected from the group consisting of glucagon-like peptide-1 (GLP-1), granulocyte colony stimulating factor (G-CSF), human growth hormone (hGH), erythropoietin (EPO), glucagon, oxyntomodulin, insulin, growth hormone-releasing hormone, growth hormone-releasing peptide, interferons, interferon receptors, G-protein-coupled receptor, interleukins, interleukin receptors, enzymes, interleukin-binding protein, cytokine-binding protein, macrophage-activating factor, macrophage peptide, B-cell factors, T-cell factors, protein A, allergy inhibitor, cell necrosis glycoprotein, immunotoxin, lymphotoxin, tumor necrosis factor, tumor suppressor, metastasis growth factor, alpha-1 antitrypsin, albumin, α-lactalbumin, apolipoprotein-E, highly-glycosylated erythropoietin, angiopoietins, hemoglobin, thrombin, thrombin receptor-activating peptide, thrombomodulin, blood factors VII, VIIa, VIII, IX and XIII, plasminogen-activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone-stimulating protein, calcitonin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factor, parathyroid hormone, relaxin, secretin, somatomedin, insulin-like growth factor, adrenocortical hormone, cholecystokinin, pancreatic polypeptide, gastrin-releasing peptide, corticotropin-releasing factor, thyroid-stimulating hormone, autotaxin, lactoferrin, myostatin, cell surface antigens, virus-derived vaccine antigens, monoclonal antibodies, polyclonal antibodies, antibody fragments and analogs thereof.

The enzymes may be selected from the group consisting of imiglucerase, iduronate 2-sulfatase, alpha-galactosidase A, iduronidase (or laronidase), alpha-glucosidase, beta-glucosidase, beta-galactosidase, galactose-6-sulfatase, acid ceramidase, acid sphingomyelinase, galactocerebrosidase, arylsulfatase A, arylsulfatase B (or galsulfase), beta-hexosaminidase A, beta-hexosaminidase B, heparin N-sulfatase, alpha-D-mannosidase, beta-glucuronidase, N-acetylgalactosamine-6 sulfatase, lysosomal acid lipase, alpha-N-acetyl-glucosaminidase (NAGLU), glucocerebrosidase, butyrylcholinesterase, chitinase, glutamate decarboxylase, lipase, uricase, platelet-activating factor acetylhydrolase, neutral endopeptidase, myeloperoxidase, acetyl-CoA-glucosaminide N-acetyltransferase, N-acetylglucosamine-6-sulfatase, galactosamine 6-sulfatase (GALN), hyaluronidase, α-fucosidase, β-mannosidase, α-neuraminidase (sialidase), N-acetyl-glucosamine-1-phosphotransferase, mucolipin-1, α-N-acetyl-galactosaminidase, N-aspartyl-β-glucosaminidase, LAMP-2, cystinosin, sialin, ceramidase, acid-β-glucosidase, galactosylceramidase, NPC1, cathepsin A (protective protein), SUMF-1, lysosomal acid lipase (LIPA), and tripeptidyl peptidase 1, but are not particularly limited thereto.

In addition, the physiologically active polypeptides as used herein refer to not only native physiologically active polypeptides, but also polypeptides having the same *in vivo* function as each polypeptide, which are analogs of each polypeptide, and such polypeptides are meant to include agonists, precursors, derivatives, fragments, or variants.

Herein, examples of insulin analogs include all those disclosed in Korean Patent Application Publication Nos. 10-2016-0007295 and 10-2017-0026284, examples of oxyntomodulin derivatives include all those disclosed in Korean Patent Application Publication No. 10-2012-0137271, and examples of insulin-releasing peptide derivatives include those disclosed in Korean Patent Application Publication No. 10-2009-0008151, but are not limited thereto. In addition, examples of enzymes include all those disclosed in International Patent Laid Open Publication No. WO2017/131496, but are not limited thereto. The entire specification of the above patents is incorporated herein by reference.

As used herein, the term "immunoglobulin Fc fragment" refers to a protein that contains the heavy-chain constant of an immunoglobulin, excluding the variable regions of the heavy and light chains, the heavy-chain constant region 1 (CHI) and the light-chain constant region 1 (CL1) of the immunoglobulin. It may further include a hinge region at the heavy-chain constant region. In the present invention, the immunoglobulin Fc fragment preferably includes a CH2 domain, a CH3 domain, or both, since the binding affinity of the immunoglobulin Fc fragment for FcRn should be maintained.

Additionally, the immunoglobulin Fc region of the present invention may be an extended Fc region including a part or all of the heavy-chain constant region 1 (CHI) and/or the light-chain constant region 1 (CL1), except for the variable regions of the heavy and light chains, as long as it maintains its intrinsic binding affinity for FcRn even when it is linked to a physiologically active polypeptide via a non-peptidyl linker.

For example, the immunoglobulin Fc region of the present invention may include 1) a CH1 domain, a CH2 domain, a CH3 domain and a CH4 domain, 2) a CH1 domain and a CH2 domain, 3) a CH1 domain and a CH3 domain, 4) a CH2 domain and a CH3 domain, 5) a combination of one or two or more of a CH1 domain, a CH2 domain, a CH3 domain and a CH4 domain and an immunoglobulin hinge region (or part of the hinge region) (*e.g.*, a combination of a CH2 domain and a CH3 domain, and a hinge region or a part thereof, and a dimer of two polypeptides having the above-described combination, and 6) a dimer of each domain of the heavy-chain constant regions and the light-chain constant region.

Further, in one embodiment, the immunoglobulin Fc region may be in a dimeric form, but is not limited thereto.

Since the immunoglobulin Fc fragment is a biodegradable polypeptide that is metabolized *in vivo,* it is safe for use as a drug carrier. In addition, since the immunoglobulin Fc fragment has a molecular weight relatively smaller than the entire immunoglobulin molecule, it is beneficial in terms of the preparation, purification and yield of the conjugate. In addition, since the Fab region, which shows high non-homogeneity due to the difference in amino acid sequences between antibodies, is removed, it can be expected that the homogeneity of substances may be greatly increased and there may be a low potential for inducing serum antigenicity.

In the present invention, the immunoglobulin Fc region includes not only a native amino acid sequence, but also a sequence mutant thereof. As used herein, the amino acid sequence mutant refers to a sequence that is different from the native amino acid sequence due to a deletion, an insertion, a non-conservative or conservative substitution or a combination thereof of one or more amino acid residues. For example, in the case of IgG Fc, amino acid residues at positions 214 to 238, 297 to 299, 318 to 322 or 327 to 331, known to be important in binding, may be used as a suitable target for modification.

In addition, various mutants are possible, including mutants having a deletion of a region capable of forming a disulfide bond, a deletion of several amino acid residues at the N-terminus of a native Fc, or an addition of methionine residue to the N-terminus of a native Fc. Further, in order to eliminate effector functions, a complement-binding site, for example, a C1q-binding site, may be removed, and an antibody dependent cell mediated cytotoxicity (ADCC) site may also be removed. Techniques of preparing such sequence derivatives of the immunoglobulin Fc fragment are disclosed in International Patent Application Publication Nos. WO 97/34631 and WO 96/32478.

Amino acid exchanges in proteins and peptides, which do not generally alter the activity of molecules, are known in the art (H. Neurath, R. L. Hill, The Proteins, Academic Press, New York, 1979). The most commonly occurring exchanges are exchanges between Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly.

In some cases, the immunoglobulin Fc fragment may also be modified by phosphorylation, sulfation, acrylation, glycosylation, methylation, farnesylation, acetylation, and amidation, *etc.*

The above-described Fc mutants are those that have a biological activity identical to the Fc fragment of the present invention and have improved structural stability against heat, pH, or the like.

In addition, these Fc fragments may be obtained from native forms isolated from humans and animals including cattle, goats, pigs, mice, rabbits, hamsters, rats and guinea pigs, or may be recombinant forms or derivatives thereof, obtained from transformed animal cells or microorganisms. Herein, they may be obtained from native forms by isolating a whole immunoglobulin from the living body of humans or animals and treating it with protease. When the whole immunoglobulin is treated with papain, it is cleaved into Fab and Fc. Meanwhile, when it is treated with pepsin, it is cleaved into pF'c and F(ab)₂. These fragments may be subjected to size-exclusion chromatography to isolate Fc or pF'c.

More specifically, the immunoglobulin Fc region may be a recombinant immunoglobulin Fc region, which is a human-derived Fc region obtained from a microorganism.

In addition, the immunoglobulin Fc fragment may be in the form of having native sugar chains, increased sugar chains compared to a native form, or decreased sugar chains compared to a native form, or may be in a deglycosylated form. The increase, decrease or removal of the immunoglobulin Fc sugar chains may be performed using conventional methods, such as a chemical method, an enzymatic method and a genetic engineering method using microorganisms. Herein, the immunoglobulin Fc fragment obtained by removing sugar chains from an Fc shows a sharp decrease in binding affinity for the complement (c1q) and a decrease or loss in antibody-dependent cell-mediated cytotoxicity or complement-dependent cytotoxicity, and thus does not induce unnecessary immune responses *in vivo.* In this regard, an immunoglobulin Fc fragment in a deglycosylated or aglycosylated form may be more suitable for use as a drug carrier.

As used herein, the term "deglycosylation" refers to an enzymatic removal of sugar moieties from an Fc fragment, and the term "aglycosylation" means an unglycosylation of Fc fragment produced in prokaryotes, specifically in *E. coli.*

Meanwhile, the immunoglobulin Fc fragment may be derived from humans or animals including cattle, goats, pigs, mice, rabbits, hamsters, rats, guinea pigs, or the like, and specifically derived from humans. In addition, the immunoglobulin Fc fragment may be an Fc fragment that is derived from IgG, IgA, IgD, IgE and IgM, a combination thereof, or a hybrid thereof. Specifically, it is derived from IgG or IgM, which is the most abundantly present in the human blood, and most specifically, it is derived from IgG known to enhance the half-life of ligand-binding proteins.

Meanwhile, as used herein, the term "combination" refers to a formation of linkage between a polypeptide encoding single-chain immunoglobulin Fc fragments of the same origin and a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

As used herein, the term "hybrid" refers to the presence of two or more sequences corresponding to immunoglobulin Fc fragments of different origins in a single-chain immunoglobulin Fc fragment. In the present invention, various types of hybrids are possible. That is, domain hybrids composed of one to four domains selected from the group consisting of CH1, CH2, CH3 and CH4 of IgG Fc, IgM Fc, IgA Fc, IgE Fc and IgD Fc are possible, and they may include a hinge region.

Meanwhile, IgG can be divided into IgG1, IgG2, IgG3 and IgG4 subclasses, and a combination or a hybrid thereof may be used in the present invention. Specifically, IgG2 and IgG4 subclasses may be used, and more specifically, the Fc fragment of IgG4 having almost no effector functions such as complement dependent cytotoxicity (CDC) may be used. That is, the most preferable immunoglobulin Fc fragment for use as a drug carrier in the present invention is a human IgG4-derived aglycosylated Fc fragment. The human-derived Fc fragment is more preferable than a non-human-derived Fc fragment, which may act as an antigen in the human body and cause undesirable immune responses such as the production of a new antibody against the antigen.

In another aspect of the present invention, there is provided a conjugate of a physiologically active polypeptide-immunoglobulin Fc fragment in which the binding affinity for an Fc gamma receptor I, IIIA and/or complement 1q (C1q) is reduced by 90% or less, 80% or less, 70% or less, 60% or less, 50% or less, 40% or less, 30% or less, 20% or less, 10% or less, 5% or less, as compared to a human serum-derived immunoglobulin G or an Fc fragment thereof.

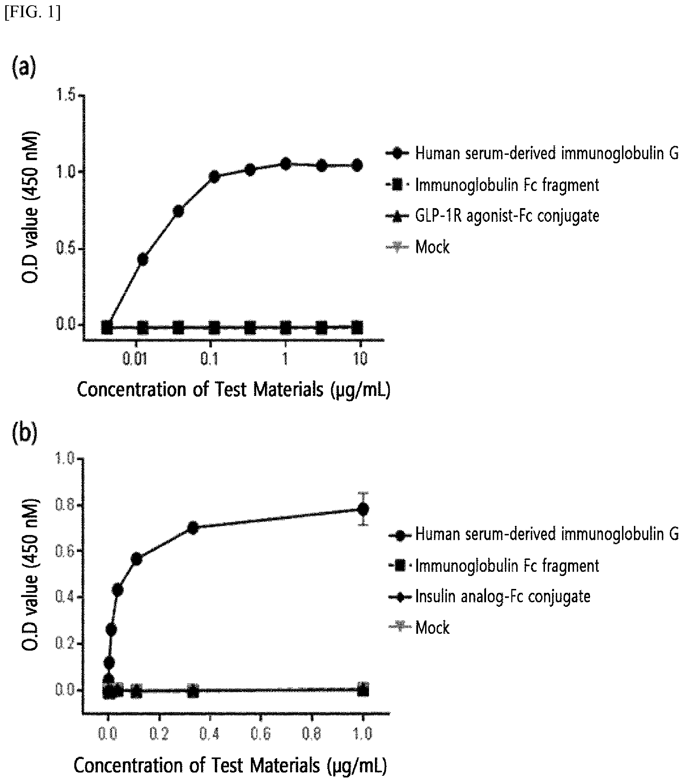

In one embodiment of the present invention, it was confirmed that it was possible to prepare conjugates which may not have an intrinsic immune effector function of an immunoglobulin Fc fragment, by linking each of insulin and a GLP-1 agonist to an immunoglobulin Fc fragment showing a significant decrease in the binding affinity for an Fc gamma receptor and a complement, via a non-peptidyl polymer (FIGS. 1, 2 and 3).

In still another aspect of the present invention, there is provided a composition for reducing an immune response, including the conjugate of the physiologically active polypeptide-immunoglobulin Fc fragment, wherein the reduction of the immune response is characterized in that the immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

The composition may remarkably reduce or counteract the immune response caused by the physiologically active polypeptide alone.

The composition may be in the form of a pharmaceutical composition.

The pharmaceutical composition of the present invention may further contain a pharmaceutically acceptable carrier, an excipient, or a diluent. The pharmaceutically acceptable carrier, excipient, or diluent may be non-naturally occurring.

As used herein, the term "pharmaceutically acceptable" refers to the property of having a sufficient amount to exhibit a therapeutic effect and not causing adverse effects, and may be easily determined by those skilled person in the art based on the factors well known in the medical field, such as the type of disease, patient's age, body weight, health status, gender, drug sensitivity, administration route, administration method, administration frequency, duration of treatment, a drug to be mixed or administered simultaneously in combination, *etc.*

The pharmaceutical composition containing the peptide of the present invention may further contain a pharmaceutically acceptable carrier. The pharmaceutically acceptable carrier may include, for oral administration, a binder, a lubricant, a disintegrant, an excipient, a solubilizing agent, a dispersant, a stabilizing agent, a suspending agent, a coloring agent, a flavoring agent, or the like.; for injections, a buffering agent, a preservative, an analgesic, a solubilizing agent, an isotonic agent, a stabilizing agent, or the like., which may be used in combination; and for topical administrations, a base, an excipient, a lubricant, a preservative, or the like, but is not particularly limited thereto.

The formulation of the composition of the present invention may be prepared in various forms by combining with a pharmaceutically acceptable carrier described above. For example, for oral administration, the composition may be formulated into tablets, troches, capsules, elixirs, suspensions, syrups, wafers, or the like. For injections, the composition may be formulated into unit-dose ampoules or multi-dose containers. The composition may also be formulated into solutions, suspensions, tablets, pills, capsules, sustained-release formulations, or the like.

Meanwhile, examples of carriers, excipients, and diluents suitable for formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil, *etc.* Additionally, the composition may further contain a filler, an anti-coagulant, a lubricant, a humectant, a flavoring agent, a preservative, *etc.*

Further, the pharmaceutical composition of the present invention may be prepared in any formulation type selected from the group consisting of tablets, pills, powders, granules, capsules, suspensions, liquid medicine for internal use, emulsions, syrups, sterile aqueous solutions, nonaqueous solvents, lyophilized formulations, and suppositories.

Furthermore, the composition may be formulated into a preparation in unit dose form suitable for administration into patient's body, specifically formulated into a preparation useful for administration of protein therapeutics according to conventional methods in the pharmaceutical field so as to be administered by an oral or parenteral route, such as through skin, intravenous route, intramuscular route, intraarterial route, intramedullar route, intrathecal route, intraventricular route, pulmonary route, transdermal route, subcutaneous route, intraperitoneal route, intranasal route, intragastrical route, topical route, sublingual route, vaginal route, or rectal route, but is not limited thereto.

Additionally, the conjugate may be used by combining with various pharmaceutically acceptable carriers approved as pharmaceutical drugs such as physiological saline or organic solvents. For increasing stability or absorptivity, carbohydrates such as glucose, sucrose, or dextran, antioxidants such as ascorbic acid or glutathione, chelating agents, low molecular weight proteins, or other stabilizers may be used as pharmaceutical drugs.

The administration dose and frequency of the pharmaceutical composition of the present invention are determined by the type of drugs, *i.e.*, active ingredients, together with various relating factors such as the disease to be treated, administration route, patient's age, gender, and body weight, and severity of the disease, *etc.*

The total effective dose of the composition of the present invention may be administered to a patient in a single dose or may be administered for a long period of time in multiple doses according to a fractionated treatment protocol. In the pharmaceutical composition of the present invention, the content of active ingredients may vary depending on the severity of the disease. Specifically, the total daily dose of the conjugate of the present invention may be about 0.0001 mg to 500 mg per 1 kg of the body weight of a patient. However, the effective dose of the conjugate is determined considering various factors including patient's age, body weight, health conditions, gender, severity of the disease, diet, and excretion rate, in addition to administration route and treatment frequency of the pharmaceutical composition. In this regard, those skilled in the art may easily determine the effective dose suitable for the particular use of the pharmaceutical composition of the present invention. The pharmaceutical composition according to the present invention is not particularly limited by the formulation and administration route and method, as long as it shows the effects of the present invention.

The pharmaceutical composition of the present invention may remarkably reduce the immunogenicity of the physiologically active polypeptide itself showing persistency and therapeutic effect *in vivo,* and thus the number and frequency of administration of the pharmaceutical preparation of the present invention can be significantly reduced, and also, a desired therapeutic effect can be achieved since undesired immune response does not occur.

In still further another aspect of the present invention, there is provided a method for preparing the conjugate of the physiologically active polypeptide-immunoglobulin Fc fragment, including:
(a) preparing a conjugate mixture of a physiologically active polypeptide-immunoglobulin Fc fragment by linking a physiologically active polypeptide to an immunoglobulin Fc fragment via a non-peptidyl linker; and
(b) separating the conjugate whose immune response is attenuated as compared to the physiologically active polypeptide or immunoglobulin Fc fragment.

The preparation method may be a method for preparing a conjugate of a physiologically active polypeptide-immunoglobulin Fc fragment, in which the immune response of the immunoglobulin Fc fragment is attenuated, including:
(a) preparing a conjugate mixture of a physiologically active polypeptide-immunoglobulin Fc fragment by linking a physiologically active polypeptide to an immunoglobulin Fc fragment with attenuated immune response non-peptidyl linker; and
(b) separating the conjugate whose immune response is reduced as compared to a serum-derived immunoglobulin G.

The preparation method may be a method for preparing a conjugate of a physiologically active polypeptide-immunoglobulin Fc fragment, in which the immune response of the immunoglobulin Fc fragment is attenuated, including:
(a) preparing a conjugate mixture of a physiologically active polypeptide-immunoglobulin Fc fragment by linking a physiologically active polypeptide to an immunoglobulin Fc fragment with attenuated immune response non-peptidyl linker; and
(b) separating the conjugate showing a decrease in the level of T-cell proliferation as compared the level of T-cell proliferation exhibited by the physiologically active polypeptide or immunoglobulin Fc fragment alone.

The preparation method may be a method for preparing a conjugate of a physiologically active polypeptide-immunoglobulin Fc fragment, in which the immune response of the immunoglobulin Fc fragment is attenuated, including:
(a) preparing a conjugate mixture of a physiologically active polypeptide-immunoglobulin Fc fragment by linking a physiologically active polypeptide to an immunoglobulin Fc fragment with attenuated immune response non-peptidyl linker; and
(b) separating the conjugate showing a decrease in the level of IL-2 secretion by T cells as compared the level of IL-2 secretion by T cells exhibited by the physiologically active polypeptide or immunoglobulin Fc fragment alone. The attenuation of the immune response is as described above.

The preparation method may be a method for preparing a conjugate of a physiologically active polypeptide-immunoglobulin Fc fragment, including:
(a) preparing a conjugate mixture of a physiologically active polypeptide-immunoglobulin Fc fragment with reduced intrinsic binding affinity for an Fc gamma receptor and a complement by linking a physiologically active polypeptide and an immunoglobulin Fc fragment via a non-peptidyl linker at both ends, respectively; wherein
(b) the binding affinity for an Fc gamma receptor I, III and/or a complement 1q (C1q) is significantly reduced as compared to a serum-derived immunoglobulin G.

The above-mentioned physiologically active polypeptide, immunoglobulin Fc fragment, non-peptidyl linker, and conjugate are as described above.

In the method of the present invention, Step (a) is a step of covalently linking a physiologically active polypeptide to an immunoglobulin Fc fragment via a non-peptidyl linker. Step (a) may include the steps of (i) linking any one of the physiologically active or polypeptide the immunoglobulin Fc fragment to a reactive group at one end of the non-peptidyl linker, and (ii) linking the remaining one to a reactive group at the other end of the non-peptidyl linker. Herein, Step (a) may further include, between steps (i) and (ii), a step of separating the physiologically active polypeptide or immunoglobulin Fc fragment linked to one end of the non-peptidyl linker. When the conjugate is prepared by this process, byproducts such as a conjugate showing a decrease in the binding affinity of the immunoglobulin Fc fragment for FcRn can be generated in addition to a conjugate that maintains the intrinsic binding affinity of the immunoglobulin Fc fragment for FcRn. For this reason, after the reaction that links the physiologically active polypeptide to the immunoglobulin Fc fragment via the non-peptidyl peptide, a process of separating the conjugate of the physiologically active polypeptide-immunoglobulin Fc fragment showing a decrease in the intrinsic binding affinity of the immunoglobulin Fc fragment for an Fc gamma receptor and a complement and a decrease in the level of T-cell proliferation and/or IL-2 secretion by T cells is additionally required.

Therefore, the method of the present invention includes a step (b) of separating the conjugate showing a significant decrease in the binding affinity for an Fc gamma receptor I, IIIA and/or complement as compared to a serum-derived immunoglobulin G, and/or a decrease in the level of T-cell proliferation and/or IL-2 secretion by T cells.

In still further another aspect of the present invention, there is provided a method for reducing the immune response of a conjugate of a physiologically active polypeptide-immunoglobulin Fc fragment by linking a physiologically active polypeptide to an immunoglobulin Fc fragment via a non-peptidyl linker, wherein the reduction of the immune response is characterized in that the immune response is attenuated as compared to (a) an immune response caused by each of the immunoglobulin Fc fragment or the physiologically active polypeptide alone; or (b) a sum of immune responses caused by each of the immunoglobulin Fc fragment or the physiologically active polypeptide alone.

The above-mentioned physiologically active polypeptide, immunoglobulin Fc fragment, non-peptidyl linker, and conjugate are as described above.

In still further another aspect of the present invention, there is provided a method for maintaining a reduced binding affinity of a conjugate of a physiologically active polypeptide-immunoglobulin Fc fragment for an Fc gamma receptor and/or a complement as compared to a human serum-derived immunoglobulin G or a fragment thereof, by linking a physiologically active polypeptide to an immunoglobulin Fc fragment, whose binding affinity for an Fc gamma receptor and a complement is removed, via a non-peptidyl linker. Herein, the maintaining of the intrinsic binding affinity may be achieved *in vitro.*

As used herein, the phrase "maintaining the binding affinity" means that even when the immunoglobulin Fc fragment, whose binding affinity for an Fc gamma receptor and/or complement is removed, is linked to a physiologically active polypeptide via a non-peptidyl linker, the binding affinity exhibited by the immunoglobulin Fc fragment, whose binding affinity for an Fc gamma receptor and/or complement is removed, for an Fc gamma receptor and/or complement which is reduced relative to a human serum-derived immunoglobulin G or a fragment thereof still exhibits a corresponding binding affinity for an Fc gamma receptor and/or complement corresponding to the binding affinity exhibited by the immunoglobulin Fc fragment itself, whose binding affinity for an Fc gamma receptor and/or complement is removed. Herein, the term "corresponding" means that it shows a difference of at least ± 20%, ± 10%, ± 5%, or 0% as compared to the binding affinity exhibited by the immunoglobulin Fc fragment itself, whose binding affinity for an Fc gamma receptor and/or complement is removed, for an Fc gamma receptor and/or complement.

Herein, the Fc gamma receptor and/or complement may be an Fc gamma receptor I, IIIA and/or complement 1q (C1q), but is not limited thereto.

The aforementioned physiologically active polypeptide, immunoglobulin Fc fragment, non-peptide linker, and conjugate are as described above.

The present invention has the advantage of effectively suppressing unnecessary immune responses that can be induced in the body due to the effector function of the immunoglobulin Fc fragment, which is resulted from the reduced binding affinity of the immunoglobulin Fc fragment an Fc gamma receptor and/or a complement, by linking the physiologically active polypeptide to the immunoglobulin Fc fragment showing a decrease in the intrinsic binding affinity for an Fc gamma receptor and/or a complement, via the non-peptidyl linker.

In still further another aspect of the present invention, there is provided a composition including the conjugate of the physiologically active polypeptide-immunoglobulin Fc fragment, in which the intrinsic binding affinity of the immunoglobulin Fc fragment for an Fc gamma receptor and/or complement thereof is significantly reduced as compared to a human serum derived-immunoglobulin G or a fragment thereof.

In still further another aspect of the present invention, there is provided a composition including the conjugate of the physiologically active polypeptide-immunoglobulin Fc fragment, in which the level of T-cell proliferation and/or IL-2 secretion by T cells is reduced as compared to the level of T-cell proliferation and/or IL-2 secretion by T cells exhibited by each of the physiologically active polypeptide or the immunoglobulin G fragment.

The aforementioned physiologically active polypeptide, immunoglobulin Fc fragment, non-peptide linker and conjugate are as described above.

The decrease in the level of T-cell proliferation and/or IL-2 secretion by T cells include all those described with respect to the aforementioned reduction or attenuation of the immune response.

Hereinafter, the the present invention will be described in more detail by way of Examples. However, these Examples are given for illustrative purposes only, and the scope of the invention is not intended to be limited by these Examples.

The long-acting conjugate used in the present invention may be prepared by binding a protein or peptide prepared by any method of the natural or recombinant origins with an immunoglobulin Fc region prepared by treating natural IgG with a specific protease or from transformed cells using recombinant technology. In the binding method used herein, the conjugate may be prepared in the form of a fusion protein in which a protein or peptide is cross-linked with the immunoglobulin Fc region using a non-peptidyl polymer.

### Preparation Example 1: Preparation of Conjugate of Immunoglobulin Fc Fragment and Physiologically Active Protein

Hereinafter, the the present invention will be described in more detail by way of Examples. However, these Examples are given for illustrative purposes only, and the scope of the invention is not intended to be limited by these Examples. The long-acting conjugate used in the present invention may be prepared by binding a protein or peptide prepared by any method of the natural or recombinant origins with an immunoglobulin Fc region prepared by treating natural IgG with a specific protease or from transformed cells using recombinant technology. In the binding method used herein, a protein or peptide may be cross-linked to an immunoglobulin Fc region using a non-peptidyl polymer, or the conjugate may be prepared in the form of a fusion protein in which a protein or peptide is linked to an immunoglobulin Fc region using recombinant technology.

### (1) Preparation of Human Immunoglobulin G4-Derived Non-Glycosylated Fc Fragment

IgG4-derived non-glycosylated Fc fragments used in the preparation of long-acting conjugates were prepared according to the method disclosed in Korean Patent Laid-Open Publication No. 10-2007-0021079 A (International Patent Laid-Open Publication No. WO2007-021129 A1).

In summary, in order to prepare IgG4-derived non-glycosylated Fc fragments used in the preparation of long-acting conjugates, Fc fragments in the form of aggregates were obtained by over-expressing the target protein using a transformed *E. coli* culture and disrupting the cells. Then, the native form of the structure was recovered after the refolding process, and then purified to obtain the final IgG4-derived non-glycosylated Fc fragments.

### (2) Preparation of Human Insulin Analog-Fc Fragment

A human insulin-analog Fc fragment was prepared according to the method disclosed in WO2014-133324 A1. As the human insulin analogs, those disclosed in the aforementioned Patent were used.

In summary, a reaction was performed to PEGylate a 3.4-kDa propion-ALD2 PEG (IDB, Korea) specifically at the N-terminus of the beta-chain of the insulin analogs. The reaction solution was purified using a cation-exchange column. In order to prepare an insulin conjugate, the purified mono-PEGylated insulin was reacted with the human immunoglobulin G4-derived non-glycosylated Fc fragment (about 50 kDa) at the amino-terminus. Herein, the reaction was performed at a pH of 6.0 to 8.2 in order to allow the insulin to specifically bind to the N-terminus of the immunoglobulin Fc. After completion of the reaction, the reaction solution was primarily purified using an anion-exchange column, and then secondarily purified using a hydrophobic column, thereby obtaining a site-specifically linked-insulin analog-Fc conjugate.

### (3) Preparation of GLP-1RAagonist-Fc Conjugate

An GLP-1R agonist-Fc conjugate was prepared according to the method disclosed in WO2008-082274 A1.

In summary, a 3.4-kDa propion-ALD2 PEG (IDB, Korea) was reacted site-specifically with the lysine residue of imidazo-acetyl-exendin-4, which is a GLP-1R agonist (glucagon-like peptide-1 receptor agonist) (CA exendin-4, Bachem, Switzerland). Then, in order to obtain a conjugate in which PEG and the GLP-1R agonist are linked at a ratio of 1:1, the reaction mixture was subjected to cation-exchange column chromatography to purify mono-PEGylated CA exendin-4. In order to prepare a GLP-1R agonist-conjugate in which the mono-PEGylated CA exendin-4 is linked specifically to the N-terminus of the immunoglobulin Fc, a reaction was performed at a pH of 5.0 to 8.2. After the coupling reaction, a two-step purification process was performed using a hydrophobic column and an anion-exchange column, thereby finally obtaining a site-specifically linked-GLP-1R agonist-Fc conjugate.

### (4) Preparation of Imiglucerase Long-Acting Conjugate

In order to link an aldehyde-polyethylene glycol (Mw=10,000 Da)-aldehyde (ALD-PEG-ALD) (SUNBRIGHT DE-100AL2, NOF CORPORATION, Japan) linker to the N-terminus of imiglucerase, imiglucerase and ALD-PEG-ALD were reacted in a 1:50 molar ratio with a concentration of imiglucerase of 1 mg/mL at 25°C for about 1 hour. Herein, the reaction was performed in the presence of 100 mM potassium phosphate at pH 6.0, and 20 mM sodium cyanoborohydride was added thereto as a reducing agent. Unreacted imiglucerase and mono-linked imiglucerase were purified by the Source 15S column (GE, USA) using a buffer containing 20 mM sodium phosphate (pH 6.0) and 2.5% (v/v) glycerol, and a sodium chloride concentration gradient.

Then, the imiglucerase linked to the purified polyethylene glycol linker was reacted with an immunoglobulin Fc fragment in a 1:50 molar ratio with a total protein concentration of 40 mg/mL at 4°C to 8°C for 12 hours to 16 hours. Herein, 100 mM potassium phosphate at pH 6.0 was used as the reaction solution, and 20 mM sodium cyanoborohydride was added thereto as a reducing agent. After completion of the reaction, the reaction solution was applied to the Source 15S column (GE, USA) using a buffer containing 10 mM sodium citrate (pH 5.0) and a sodium chloride concentration gradient and to the Protein A column (GE, USA) using a concentration gradient of a buffer containing 20 mM Tris (pH 7.5), 5% (v/v) glycerol, 100 mM sodium citrate (pH 3.7), sodium chloride, and 10% glycerol, and finally, to Superdex™ 200 column (GE, USA) using a 50 mM sodium citrate buffer (pH 6.1) containing sodium chloride, thereby purifying the conjugate in which the immunoglobulin Fc was covalently linked to imiglucerase by a polyethylene glycol linker.

Specifically, the purified non-peptidyl polymer enzyme and the immunoglobulin Fc region were covalently bonded to the unreacted aldehyde group (-CHO) on the other end of the non-peptidyl polymer and to the -NH₂ at the N-terminus of the immunoglobulin Fc region, and purified after the covalent bonding, thereby completing the preparation of the enzyme conjugate.

The finally prepared imiglucerase-polyethylene glycol linker-immunoglobulin Fc conjugate was in a form in which imiglucerase monomers were linked to one chain of the immunoglobulin Fc, which consists of two chains, by a polyethylene glycol linker.

### Experimental Example 1: Evaluation of Binding Affinity for Fc Gamma Receptor I and IIIA (FcγRI. FcγRIIIA)

In order to evaluate the binding affinity for the Fc gamma receptors at the protein level, FcγRI and FcγRIIIA proteins were obtained using a CHO (chinese hamster ovary) cell expression system. Specifically, an expression vector expressing the extracellular domain of FcγRI and FcγRIIIA and a gene encoding glutathione S-transferase (GST) tag under a cytomegalovirus promoter (CMV promoter) was prepared, and CHO cells were transformed using the expression vector. Transformed cells were selected with 1 mg/ml G418 (Geneticin, Cellgro, USA) and proliferated to induce the expression of Fc gamma receptors in a serum-free medium. The FcγRI and FcγRIIIA proteins were purified using a GST-specific column.

### Experimental Example 2: Evaluation of Binding Affinity for Fc Gamma Receptor I (FcγRI)

The FcγRI was diluted to a concentration of 1.5 µg/mL in a 50 mM sodium carbonate buffer (pH 9.0) and coated onto a 96-well plate (4°C, 16 hours) for the enzyme-linked immunosorbent assay (ELISA). After washing three times, in order to inhibit a non-specific protein binding, D-PBS (dulbecco's phosphate buffered saline) containing 1% gelatin was added thereto, allowed to stand at 37°C for 1 hour, and then removed. Human serum-derived immunoglobulin G, the immunoglobulin Fc fragment prepared in Preparation Example 1; and insulin analog-Fc conjugate and GLP-1R agonist-Fc conjugate, which were physiologically active protein-Fc conjugates, were subjected to a 3-fold serial dilution from 10 µg/mL or 1 µg/mL and added to a 96-well plate. The reaction solution (D-PBS containing 1% gelatin) alone was used as a mock sample. Thereafter, the proteins were cultured at room temperature for 2 hours to induce a binding reaction with FcyRI. In order to detect the amount of the immunoglobulin G, immunoglobulin Fc and conjugates bound to FcγRI, an anti-human immunoglobulin G antibody conjugated with horseradish peroxidase was added and allowed to bind thereto (room temperature, 2 hour), and then TMB (3,3',5,5'-tetramethylbenzidine) substrate was added for color development. Subsequently, the reaction was terminated by adding 2N HCl, and the absorbance at 450 nm was measured.

As a result, as can be confirmed in FIG. 1 (a) and FIG. 1 (b), the immunoglobulin Fc fragment of the present invention, the insulin analog-Fc conjugate, and the GLP-1R agonist-Fc conjugate showed a significantly reduced binding affinity for FcγRI as compared to the human serum-derived immunoglobulin. These results imply that the conjugates of the present invention do not induce an unnecessary immune response because they almost have no binding affinity for FcγRI, which causes an immune response, even when administered to the human body.

### Experimental Example 3: Evaluation of Binding Affinity for Fc Gamma Receptor IIIA (FcγRIIIA)

Human serum-derived immunoglobulin G, the immunoglobulin Fc fragment prepared in Preparation Example 1; and insulin analog-Fc conjugate and GLP-1R agonist-Fc conjugate, which are physiologically active protein-Fc conjugates, were subjected to a 3-fold serial dilution from 9 µg/mL using a 50 mM sodium carbonate buffer (pH 9.0) and coated onto a 96-well plate (4°C, 16 hours) for the enzyme-linked immunosorbent assay (ELISA). After washing three times, in order to inhibit a non-specific protein binding, D-PBS containing 5% skim milk powder was added thereto, allowed to stand at 37°C for 1 hour, and then removed. The FcγRIIIA was diluted to a concentration of 1 µg/mL and then cultured at room temperature for 2 hours to induce a binding reaction with the human serum-derived immunoglobulin G serum, immunoglobulin Fc fragment prepared in Preparation Example 1; and insulin analog-Fc conjugate and GLP-1R agonist-Fc conjugate, which are physiologically active protein-Fc conjugates. In order to detect the amount of bound FcγRIIIA, a rabbit-derived anti-GST antibody was added and allowed to react with FcγRIIIA. Thereafter, an anti-rabbit immunoglobulin G antibody conjugated with peroxidase was added and allowed to bind thereto (room temperature, 2 hours each), and then TMB (,3',5,5'-tetramethylbenzidine) substrate was added for color development. Subsequently, the reaction was terminated by adding 2N HCl, and the absorbance at 450 nm was measured.

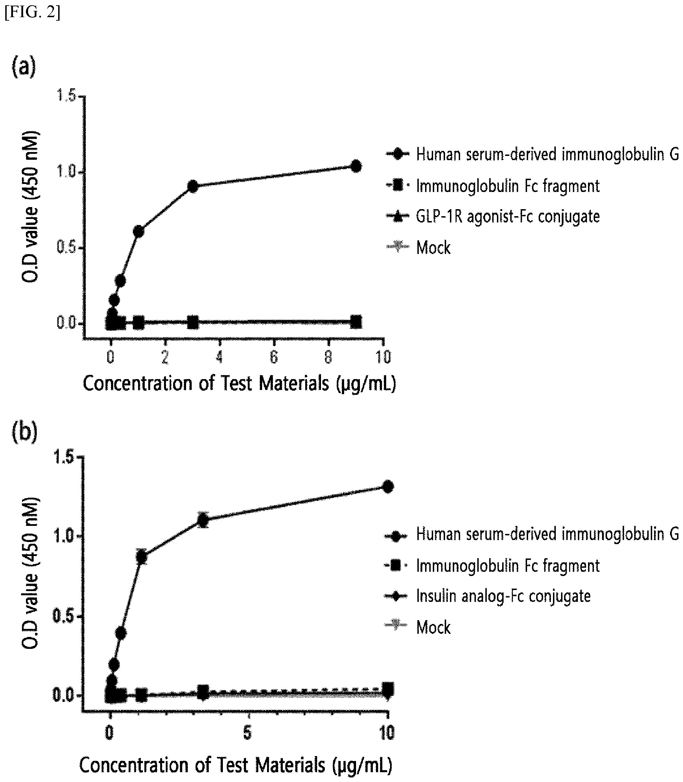

As a result, as shown in FIG. 2 (a) and FIG. 2 (b), the immunoglobulin Fc fragment of the present invention, the insulin analog-Fc conjugate, and the GLP-1R agonist-Fc conjugate showed a significantly reduced binding affinity for FcγRIIIA as compared to the human serum-derived immunoglobulin. These results imply that the conjugates of the present invention do not induce an unnecessary immune response because they almost have no binding affinity for FcγRIIIA, which causes an immune response, even when administered to the human body.

### Experimental Example 4: Evaluation of Binding Affinity for Complement 1q(C1q)

Human serum-derived immunoglobulin G, the immunoglobulin Fc fragment prepared in Preparation Example 1; and insulin analog-Fc conjugate and GLP-1R agonist-Fc conjugate, which are physiologically active protein-Fc conjugates, were subjected to a 3-fold serial dilution from 9 µg/mL or a 2-fold serial dilution from 10 µg/mL using a 50 mM sodium carbonate buffer (pH 9.0) and coated onto a 96-well plate (4°C, 16 hours) for the enzyme-linked immunosorbent assay (ELISA). After washing three times, in order to inhibit a non-specific protein binding, D-PBS containing 1% gelatin was added thereto, allowed to stand at 37°C for 1 hour, and then removed. The C1q (Quidel, USA) was diluted to a concentration of 4 µg/mL and then cultured at room temperature for 2 hours to induce a binding reaction with the human serum-derived immunoglobulin G serum, immunoglobulin Fc fragment prepared in Preparation Example 1; and insulin analog-Fc conjugate and GLP-1R agonist-Fc conjugate, which are physiologically active protein-Fc conjugates. In order to detect the amount of bound C1q, an anti-human C1q antibody conjugated with peroxidase was added and allowed to bind to thereto (room temperature, 2 hours each). Then, a TMB substrate was added for color development. Subsequently, the reaction was terminated by adding 2N HCl, and the absorbance at 450 nm was measured.

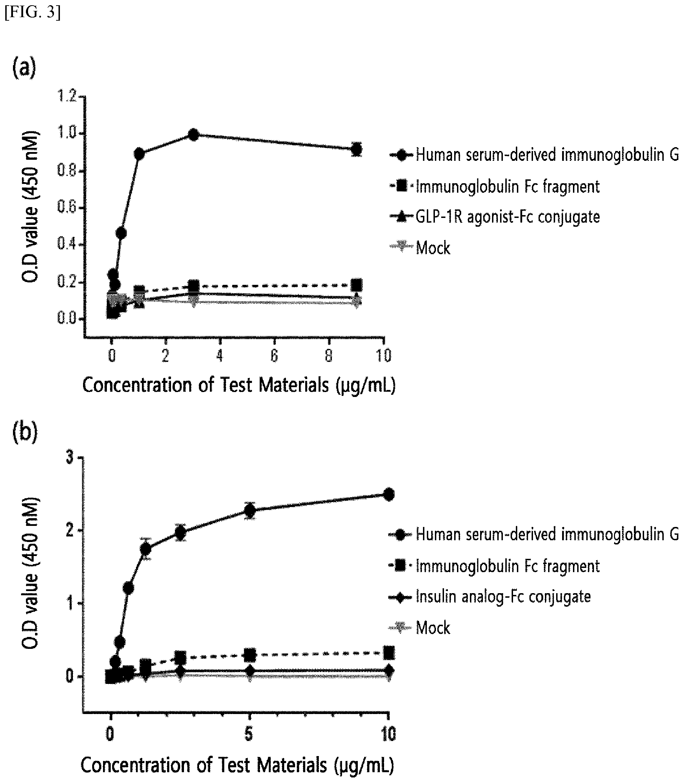

As a result, as shown in FIG. 3 (a) and FIG. 3 (b), the immunoglobulin Fc fragment of the present invention, the insulin analog-Fc conjugate, and the GLP-1R agonist-Fc conjugate showed a significantly reduced binding affinity for C1q as compared to the human serum-derived immunoglobulin. These results imply that the conjugates of the present invention do not induce an unnecessary immune response, for example, they have a low risk of inducing an immune response such as cytotoxicity, inflammation or the like, because they almost have no binding affinity for C1q, which causes an immune response, even when administered to the human body.

### Experimental Example 5: Confirmation of Immunogenicity by EpiScreen Assay

In order to confirm the immunogenicity of the insulin analog, immunoglobulin Fc and insulin analog-immunoglobulin globulin Fc conjugate, the EpiScreen assay was used to measure the immunogenicity *ex vivo* by quantifying T cell responses for protein therapeutics. In particular, the frequency of T cell proliferation and the degree of IL-2 secretion were observed.

For the EpiScreen assay, peripheral blood mononuclear cells (PBMC) were isolated from the blood of donors to be used, and the cells were incubated and maintained in AIM-V medium (Invitorogen). PBMCs isolated from a total of 50 donors were used for the test.

In order to confirm the frequency of T-cell proliferation, PBMCs isolated from each donor were diluted in the medium to a concentration of 4 to 6 × 10⁶ cells/mL and inoculated at 1 mL/well into a 24-well plate. The insulin analog was diluted to a proper concentration so that the final concentration was 5 µM, and the insulin analog-immunoglobulin Fc conjugate, immunoglobulin Fc, humanized-A33 and KLH (Keyhole limpet haemocyanin) were diluted to a final concentration of 0.3 µM and added to the 24-well plate containing PBMC at 0.5 mL per well. The cells were cultured for a total of 8 days at 37°C under 5% CO₂. On day 5, day 6, day 7, and day 8, the cells in each well were resuspended and transferred to a 96-well plate at 100 µL per 3 wells (n = 3). ³H-thymidine (Perkin Elmer) was diluted with 0.75 µCi in the AIM-V medium, added to the 96-well at 100 µL per well and then incubated for 18 hours. The cells were transferred to a 96-well filter with a cell harvester, and counts per minute (CPM) was read on a microbeta counter using a Meltilex (Perkin Elmer).

The degree of IL-2 secretion was measured by ELISpot. ELISpot plates were coated with IL-2 capture antibodies overnight and then washed three times with PBS (phosphate buffered saline). After keeping it overnight with a blocking buffer (1% bovine serum albumin/PBS), the plates were washed with the AIM-V medium. 50 µL of AIM-V medium was added to each well, and 100 µL of PBMC diluted to a concentration of 4 to 6 × 10⁶ cells/mL in the medium was added thereto. The insulin analog was diluted to a proper concentration so that the final concentration is 5 µM, and the insulin analog-immunoglobulin Fc conjugate, immunoglobulin Fc, humanized-A33 and KLH (Keyhole limpet haemocyanin) were diluted to a final concentration of 0.3 µM and inoculated in the proper wells at n = 6. After incubation for 8 days, the cells were washed once with distilled water and three times with PBS. Biotin-labeled IL-2 antibodies were then reacted at 37°C for 1.5 hours. After washing three times with PBS, streptavidin-AP (alkaline phosphate) was reacted at room temperature for 1.5 hours and then further washed three times with PBS. Thereafter, BCIP/NBT (5-bromo-4-chloro-3-indolyl phosphate/Nitro blue tetrazolium) was added as a substrate to allowed for a color reaction for 30 minutes, and then the reaction was terminated by washing with distilled water. Then, the plates were dried and scanned using an Immunoscan Analyzer, and spots per well (spw) was analyzed using the Immunoscan Software (Version 5).

The frequency of T-cell proliferation and the degree of IL-2 secretion were analyzed by calculating SI (Stimulating index). The results were judged as positive when SI was 2 or higher.

Table 1 below shows the frequency of *Ex vivo* T cell proliferation of the insulin analog, which is a physiologically active polypeptide, and immunoglobulin Fc alone, insulin analog-immunoglobulin Fc-conjugate, and humanized-A33 antibody and KLH (Keyhole limpet haemocyanin), which are positive control.

**[Table 1]**

| Frequency of T-cell proliferation | Average SI | Standard deviation | Reaction frequency (%) |
|---|---|---|---|
| Insulin analog | 2.28 | 0.49 | 12 |
| Insulin analog-immunoglobulin Fc-conjugate | 2.02 | 0.06 | 4 |
| Immunoglobulin Fc | 2.32 | 0.45 | 4 |
| Humanized-A33 antibody | 2.42 | 0.65 | 24 |
| KLH | 5.18 | 4.00 | 92 |

Table 2 below shows the degree of IL-2 secretion by *Ex vivo* T cells of the insulin analog, which is a physiologically active polypeptide, and immunoglobulin Fc alone, insulin analog-immunoglobulin Fc-conjugate, and humanized-A33 antibody and KLH (Keyhole limpet haemocyanin), which are positive control.

**[Table 2]**

| Degree of IL-2 secretion by T cells | Average SI | Standard deviation | Reaction frequency (%) |
|---|---|---|---|
| Insulin analog | 2.10 | 0.49 | 12 |
| Insulin analog-immunoglobulin Fc-conjugate | N/A | N/A | 0 |
| Immunoglobulin Fc | 2.16 | 0.28 | 4 |
| Humanized-A33 antibody | 2.25 | 0.42 | 32 |
| KLH | 3.91 | 1.84 | 88 |

As can be seen from the results of Tables 1 and 2, the conjugate of the physiologically active polypeptide-immunoglobulin Fc fragment of the present invention significantly reduces the immune response triggered by the physiologically active polypeptide or immunoglobulin Fc alone, each of which is a constitutional element. Moreover, these results suggest that since the physiologically active polypeptide and the immunoglobulin Fc fragment form a conjugate via a non-peptidyl linker, it is capable of counteracting an unnecessary immune response that can be triggered by the physiologically active polypeptide, an API. In addition, the results also imply that the conjugate can be used as a therapeutic agent without an excessive immune response *in vivo* with increased serum half-life due to the binding of the Fc fragment, which generally increases serum persistence.

### Experimental Example 6: Confirmation of Therapeutic Effect of Long-Acting Conjugate

### Confirmation of Binding Ability of Imiglucerase Long-Acting Conjugate for M6P Receptor

In order to confirm the binding affinity for M6PR, the binding affinity of imiglucerase (control group) and the imiglucerase long-acting conjugate (experimental group) prepared above was confirmed using SPR (surface plasmon resonance, BIACORE T200). The M6PR was purchased from Rnd systems. The experiment was carried out as follow: M6PR was immobilized on a CM5 chip using an amine binding method, and the control group was allowed to flow into M6PR at a concentration ranging from 100 nM to 6.24 nM and the experimental group was allowed to flow into M6PR at a concentration ranging from 200 nM and 12.5 nM to confirm the binding affinity.

Hepes buffer (HBS-EP) at pH 7.5 was used as the running buffer. All test materials were diluted with the running buffer to induce binding, and dissociation was also carried out using the running buffer. The test materials were allowed to flow into M6PR immobilized on the chip for 10 minutes to induce binding, and dissociation was carried out for 6 minutes.

Subsequently, 5mM NaOH/50mM NaCl was allowed to flow for about 30 seconds into the conjugate bound to M6PR in order to bind different concentrations of the control or experimental group. The binding affinity between M6PR and imiglucerase or the imiglucerase long-acting conjugate was analyzed using the BIAevaluation program. Kₐ(association rate constant), K_{d}(dissociation rate constant) and K_{D}(affinity constant) were calculated using a 1:1 Langmuir binding model.

The results confirming the activity of the imiglucerase long-acting conjugate of the present invention can be summarized as follows: the following values indicate the activity of the long-acting conjugate relative to the activity of imiglucerase (100%).

**[Table 3]**

| *In vitro* Activity (vs. 1^{st} ERT) | |
|---|---|
| Enzyme Activity | M6PR Binding Affinity |
| 93% | 22% |

While the present invention has been described with reference to the particular illustrative embodiments, it will be understood by those skilled in the art to which the present invention pertains that the present invention may be embodied in other specific forms without departing from the technical spirit or essential characteristics of the present invention. Therefore, the embodiments described above are considered to be illustrative in all respects and not restrictive. Furthermore, the scope of the present invention is defined by the appended claims rather than the detailed description, and it should be understood that all modifications or variations derived from the meanings and scope of the present invention and equivalents thereof are included in the scope of the appended claims

## Claims

1. A conjugate of a physiologically active polypeptide-immunoglobulin Fc fragment having the following features of (a), (b), or both, in which a physiologically active polypeptide is linked to an immunoglobulin Fc fragment whose immune response is attenuated as compared to a human serum derived-immunoglobulin G or a fragment thereof: wherein
(a) the conjugate exhibits an attenuated immune response compared to an immune response caused by the physiologically active polypeptide alone; and
(b) the conjugate exhibits a corresponding or reduced immune response compared to an immune response of the immunoglobulin Fc fragment itself.

2. The conjugate of claim 1, wherein the immune response is triggered by T-cell proliferation or secretion of IL-2 (Interleukin-2) by T cells of the immunoglobulin Fc fragment, physiologically active polypeptide, or conjugate of the physiologically active polypeptide-immunoglobulin Fc fragment.

3. The conjugate of claim 1, wherein the immunoglobulin Fc fragment has a reduced binding affinity for an Fc gamma receptor by 90% or less as compared to a human serum-derived immunoglobulin G or an Fc fragment thereof.

4. The conjugate of claim 1, wherein the immunoglobulin Fc fragment has a reduced binding affinity for a complement 1q by 90% or less as compared to a human serum-derived immunoglobulin G or an Fc fragment thereof.

5. The conjugate of claim 1, wherein the conjugate of the physiologically active polypeptide-immunoglobulin Fc fragment exhibits a reduced T cell proliferation by 90% or less as compared to the physiologically active polypeptide.

6. The conjugate of claim 1, wherein the conjugate of the physiologically active polypeptide-immunoglobulin Fc fragment exhibits a reduced secretion of IL-2 (Interleukin-2) by T cells by 90% or less as compared to the physiologically active polypeptide.

7. The conjugate of claim 1, wherein the physiologically active polypeptide and the immunoglobulin Fc fragment are linked via a non-peptidyl linker.

8. The conjugate of claim 7, wherein the non-peptidyl linker is selected from the group consisting of polyethylene glycol, polypropylene glycol, an ethylene glycol-propylene glycol copolymer, polyoxytheylated polyol, polyvinyl alcohol, polysaccharide, dextran, polyvinyl ether, a biodegradable polymer, a lipid-polymer, chitin, hyaluronic acid, and a combination thereof.

9. The conjugate of claim 7, wherein the non-peptidyl linker is a polyethylene glycol polymer represented by Chemical Formula 1 below: 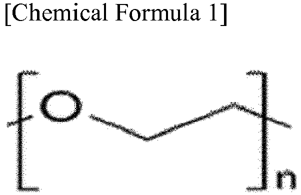 wherein, n= 10 to 2400.

10. The conjugate of claim 1, wherein the physiologically active polypeptide is a hormone, a cytokine, an interleukin, an interleukin-binding protein, an enzyme, an antibody, a growth factor, a transcription factor, a blood factor, a vaccine, a structural protein, a ligand protein or a receptor, a cell surface antigen, or a receptor antagonist.

11. The conjugate of claim 1, wherein the physiologically active polypeptide is selected from the group consisting of glucagon-like peptide-1 (GLP-1), granulocyte colony stimulating factor (G-CSF), human growth hormone (hGH), erythropoietin (EPO), glucagon, oxyntomodulin, insulin, growth hormone-releasing hormone, growth hormone-releasing peptide, interferons, interferon receptors, G-protein-coupled receptor, interleukins, interleukin receptors, enzymes, interleukin-binding protein, cytokine-binding protein, macrophage-activating factor, macrophage peptide, B-cell factors, T-cell factors, protein A, allergy inhibitor, cell necrosis glycoprotein, immunotoxin, lymphotoxin, tumor necrosis factor, tumor suppressor, metastasis growth factor, alpha-1 antitrypsin, albumin, α-lactalbumin, apolipoprotein-E, highly-glycosylated erythropoietin, angiopoietins, hemoglobin, thrombin, thrombin receptor-activating peptide, thrombomodulin, blood factors VII, VIIa, VIII, IX and XIII, plasminogen-activating factor, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone growth factor, bone-stimulating protein, calcitonin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factor, parathyroid hormone, relaxin, secretin, somatomedin, insulin-like growth factor, adrenocortical hormone, cholecystokinin, pancreatic polypeptide, gastrin-releasing peptide, corticotropin-releasing factor, thyroid-stimulating hormone, autotaxin, lactoferrin, myostatin, cell surface antigens, virus-derived vaccine antigens, monoclonal antibodies, polyclonal antibodies, antibody fragments and analogs thereof.

12. The conjugate of claim 1, wherein the immunoglobulin Fc fragment comprises a CH2 domain, a CH3 domain, or both.

13. The conjugate of claim 1, wherein the immunoglobulin Fc fragment is non-glycosylated.

14. The conjugate of claim 1, wherein the immunoglobulin Fc fragment further comprises a hinge region.

15. The conjugate of claim 1, wherein the immunoglobulin Fc fragment is selected from the group consisting of IgG, IgA, IgD, IgE, IgM, a combination thereof and a hybrid thereof.

16. The conjugate of claim 1, wherein the immunoglobulin Fc fragment is an IgG4 Fc fragment.

17. The conjugate of claim 1, wherein
(a) an immune response caused by the physiologically active polypeptide alone comprises T cell proliferation, secretion of IL-2 by T cells or both, and the conjugate exhibits an attenuated immune response compared to an immune response caused by the physiologically active polypeptide alone; and
(b) the immunoglobulin Fc fragment itself exhibits a reduced binding affinity for an Fc gamma receptor, complement, or both, as compared to an immunoglobulin G or an Fc fragment thereof, and the conjugate maintains the reduced binding affinity of such immunoglobulin Fc fragment.

18. A composition for reducing the immune response, comprising the conjugate of the physiologically active polypeptide-immunoglobulin Fc fragment of any one of claims 1 to 17,
wherein the reduction of the immune response is **characterized in that** the immune response is attenuated as compared to an immune response caused by each of the immunoglobulin Fc fragment or the physiologically active polypeptide alone.

19. A method for preparing the conjugate of the physiologically active polypeptide-immunoglobulin Fc fragment of any one of claims 1 to 17, comprising:
(a) preparing a conjugate mixture of a physiologically active polypeptide-immunoglobulin Fc fragment by linking a physiologically active polypeptide to an immunoglobulin Fc fragment via a non-peptidyl linker; and
(b) separating the conjugate whose immune response is attenuated as compared to the physiologically active polypeptide or immunoglobulin Fc fragment.

20. A method for preparing the conjugate of the physiologically active polypeptide-immunoglobulin Fc fragment of any one of claims 1 to 17, comprising:
(a) preparing a conjugate mixture of a physiologically active polypeptide-immunoglobulin Fc fragment by linking a physiologically active polypeptide to an immunoglobulin Fc fragment with an attenuated immune response via a non-peptidyl linker; and
(b) separating the conjugate whose immune response is attenuated as compared to a serum-derived immunoglobulin G.

21. The method of claim 20, wherein the attenuation in the immune response is **characterized in that** the binding affinity of the immunoglobulin Fc fragment for an Fc gamma receptor and a complement is removed.

22. The method of claim 20, wherein the immunoglobulin Fc fragment whose immune response is attenuated is a non-glycosylated Fc fragment.

23. The method of claim 20, wherein Step (b) is for separating a conjugate in a form in which the non-peptidyl linker is linked to the N-terminus of the immunoglobulin Fc fragment.

24. A method for reducing an immune response of a conjugate of a physiologically active polypeptide-immunoglobulin Fc fragment by linking a physiologically active polypeptide to an immunoglobulin Fc fragment via a non-peptidyl linker,
wherein the reduction of the immune response is **characterized in that** the immune response is attenuated as compared to an immune response caused by each of the immunoglobulin Fc fragment or the physiologically active polypeptide alone.

25. The method of claim 24, wherein the immune response is triggered by T-cell proliferation of the immunoglobulin Fc fragment, physiologically active polypeptide, or conjugate of the physiologically active polypeptide-immunoglobulin Fc fragment, or secretion of IL-2 (Interleukin-2) by T cells.

26. A method for maintaining a reduced binding affinity of a conjugate of a physiologically active polypeptide-immunoglobulin Fc fragment for an Fc gamma receptor and a complement as compared to a human serum-derived immunoglobulin G or a fragment thereof, by linking a physiologically active polypeptide to an immunoglobulin Fc fragment, whose binding affinity for an Fc gamma receptor and a complement is removed, via a non-peptidyl linker.

27. The method of claim 26, wherein the immunoglobulin Fc fragment, whose binding affinity for an Fc gamma receptor and a complement is removed, is a non-glycosylated Fc fragment.

28. A composition comprising the conjugate of the physiologically active polypeptide-immunoglobulin Fc fragment of any one of claims 1 to 17, in which the intrinsic binding affinity of the immunoglobulin Fc fragment for an Fc gamma receptor or complement is reduced as compared to an immunoglobulin G or a fragment thereof.

29. A composition comprising the conjugate of the physiologically active polypeptide-immunoglobulin Fc fragment of any one of claims 1 to 17, in which T-cell proliferation or secretion of IL-2 by T cells of the physiologically active polypeptide, or both is reduced.
